# EUROPEAN PATENT APPLICATION

(11) **EP 3 524 616 A1**
(43) Date of publication of application: **14.08.2019**
(21) Application number: 19159653.5
(22) Date of filing: 30.04.2015
(51) Int. Cl.: C07K 14/735, C07K 16/28, C12N 5/0783

(54) **CS1 SPECIFIC MULTI-CHAIN CHIMERIC ANTIGEN RECEPTOR**

(30) Priority: 02.05.2014 US 201461987805 P
(62) Divisional of application: 15720694.7
(71) Applicant: Cellectis, 75013 Paris (FR)
(72) Inventor: GALETTO, Roman, 75014 Paris (FR)
(74) Representative: Zacco Denmark A/S

(57) **Abstract**

The present invention relates to a new generation of chimeric antigen receptors (CAR) referred to as multi-chain CARs, which are made specific to the antigen CS1. Such CARs aim to redirect immune cell specificity and reactivity toward malignant cells expressing the tumor antigen CS1. The alpha, beta and gamma polypeptides composing these CARs are designed to assemble in juxtamembrane position, which forms flexible architecture closer to natural receptors, that confers optimal signal transduction. The invention encompasses the polynucleotides, vectors encoding said multi-chain CAR and the isolated cells expressing them at their surface, in particularly for their use in immunotherapy. The invention opens the way to efficient adoptive immunotherapy strategies for treating cancer, especially multiple myeloma.

SEQUENCE LISTING

<110> CELLECTIS

<120> CS1 SPECIFIC MULTI-CHAIN CHIMERIC ANTIGEN RECEPTOR

<130> P81501739EP01

<160> 22

<170> PatentIn version 3.5

<210> 1

<211> 25
<212> PRT
<213> homo sapiens

<220>
<223> Fc Receptor for IgE, alpha chain, signal peptide

<400> 1
Met Ala Pro Ala Met Glu Ser Pro Thr Leu Leu Cys Val Ala Leu Leu
1               5                   10                  15

Phe Phe Ala Pro Asp Gly Val Leu Ala
            20                  25

<210> 2

<211> 45
<212> PRT
<213> homo sapiens

<220>
<223> CD8a-hinge

<400> 2
Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp
        35                  40                  45

<210> 3

<211> 15
<212> PRT
<213> artificial sequence
<220>
<223> description of artificial sequence: synthetic oligopeptide

<220>
<223>  linker for VH and VL fragments of the scFv

<400>  3
Gly Gly Gly Gly Ser Gly Gly Gly Gly Ser Gly Gly Gly Gly Ser
1               5                   10                  15


<210>  4

<211>  52
<212>  PRT
<213>  homo sapiens

<220>
<223>  Fc Receptor for IgE, alpha chain, transmembrane and intracellular
domain

<400>  4
Phe Phe Ile Pro Leu Leu Val Val Ile Leu Phe Ala Val Asp Thr Gly
1               5                   10                  15


Leu Phe Ile Ser Thr Gln Gln Gln Val Thr Phe Leu Leu Lys Ile Lys
            20                  25                  30


Arg Thr Arg Lys Gly Phe Arg Leu Leu Asn Pro His Pro Lys Pro Asn
        35                  40                  45


Pro Lys Asn Asn
    50


<210>  5

<211>  215
<212>  PRT
<213>  homo sapiens

<220>
<223>  Fc Receptor for IgE, beta chain, without ITAM

<400>  5
Met Asp Thr Glu Ser Asn Arg Arg Ala Asn Leu Ala Leu Pro Gln Glu
1               5                   10                  15


Pro Ser Ser Val Pro Ala Phe Glu Val Leu Glu Ile Ser Pro Gln Glu
            20                  25                  30


Val Ser Ser Gly Arg Leu Leu Lys Ser Ala Ser Ser Pro Pro Leu His
        35                  40                  45


Thr Trp Leu Thr Val Leu Lys Lys Glu Gln Glu Phe Leu Gly Val Thr
    50                  55                  60


Gln Ile Leu Thr Ala Met Ile Cys Leu Cys Phe Gly Thr Val Val Cys

65                  70                  75                  80

Ser Val Leu Asp Ile Ser His Ile Glu Gly Asp Ile Phe Ser Ser Phe
                85                  90                  95

Lys Ala Gly Tyr Pro Phe Trp Gly Ala Ile Phe Phe Ser Ile Ser Gly
            100                 105                 110

Met Leu Ser Ile Ile Ser Glu Arg Arg Asn Ala Thr Tyr Leu Val Arg
        115                 120                 125

Gly Ser Leu Gly Ala Asn Thr Ala Ser Ser Ile Ala Gly Gly Thr Gly
    130                 135                 140

Ile Thr Ile Leu Ile Ile Asn Leu Lys Lys Ser Leu Ala Tyr Ile His
145                 150                 155                 160

Ile His Ser Cys Gln Lys Phe Phe Glu Thr Lys Cys Phe Met Ala Ser
                165                 170                 175

Phe Ser Thr Glu Ile Val Val Met Met Leu Phe Leu Thr Ile Leu Gly
            180                 185                 190

Leu Gly Ser Ala Val Ser Leu Thr Ile Cys Gly Ala Gly Glu Glu Leu
        195                 200                 205

Lys Gly Asn Lys Val Pro Glu
    210                 215

<210>  6

<211>  42
<212>  PRT
<213>  homo sapiens

<220>
<223>  41BB Intracellular domain

<400>  6
Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  7

<211>  41
<212>  PRT
<213>  homo sapiens

<220>
<223>  CD28 Intracellular domain

<400>  7
Arg Ser Lys Arg Ser Arg Gly Gly His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  8

<211>  18
<212>  PRT
<213>  homo sapiens

<220>
<223>  Fc Receptor for IgE, gamma chain, signal peptide

<400>  8
Met Ile Pro Ala Val Val Leu Leu Leu Leu Leu Leu Val Glu Gln Ala
1               5                   10                  15

Ala Ala

<210>  9

<211>  43
<212>  PRT
<213>  homo sapiens

<220>
<223>  Fc Receptor for IgE, gamma chain, without ITAM

<400>  9
Leu Gly Glu Pro Gln Leu Cys Tyr Ile Leu Asp Ala Ile Leu Phe Leu
1               5                   10                  15

Tyr Gly Ile Val Leu Thr Leu Leu Tyr Cys Arg Leu Lys Ile Gln Val
            20                  25                  30

Arg Lys Ala Ala Ile Thr Ser Tyr Glu Lys Ser
        35                  40

<210>  10

<211>  112

<212>  PRT
<213>  homo sapiens

<220>
<223>  CD3z intracellular domain

<400>  10
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  11

<211>  22
<212>  PRT
<213>  artificial sequence

<220>
<223>  description of artificial sequence: synthetic oligopeptide

<220>
<223>  GSG-P2A ribosomal skip peptide

<400>  11
Gly Ser Gly Ala Thr Asn Phe Ser Leu Leu Lys Gln Ala Gly Asp Val
1               5                   10                  15

Glu Glu Asn Pro Gly Pro
            20

<210>  12

<211>  21

<212>  PRT
<213>  artificial sequence

<220>
<223>  description of artificial sequence: synthetic oligopeptide

<220>
<223>  GSG-T2A ribosomal skip peptide

<400>  12
Gly Ser Gly Glu Gly Arg Gly Ser Leu Leu Thr Cys Gly Asp Val Glu
1               5                   10                  15

Glu Asn Pro Gly Pro
            20

<210>  13

<211>  119
<212>  PRT
<213>  artificial sequence

<220>
<223>  description of artificial sequence: synthetic oligopeptide

<220>
<223>  anti-CS1 Luc63 heavy chain variable region

<400>  13
Glu Val Lys Leu Leu Glu Ser Gly Gly Gly Leu Val Gln Pro Gly Gly
1               5                   10                  15

Ser Leu Lys Leu Ser Cys Ala Ala Ser Gly Phe Asp Phe Ser Arg Tyr
            20                  25                  30

Trp Met Ser Trp Val Arg Gln Ala Pro Gly Lys Gly Leu Glu Trp Ile
        35                  40                  45

Gly Glu Ile Asn Pro Asp Ser Ser Thr Ile Asn Tyr Thr Pro Ser Leu
    50                  55                  60

Lys Asp Lys Phe Ile Ile Ser Arg Asp Asn Ala Lys Asn Thr Leu Tyr
65                  70                  75                  80

Leu Gln Met Ser Lys Val Arg Ser Glu Asp Thr Ala Leu Tyr Tyr Cys
                85                  90                  95

Ala Arg Pro Asp Gly Asn Tyr Trp Tyr Phe Asp Val Trp Gly Ala Gly
            100                 105                 110

Thr Thr Val Thr Val Ser Ser
        115

<210>  14

<211>  107
<212>  PRT
<213>  artificial sequence

<220>
<223>  description of artificial sequence: synthetic oligopeptide

<220>
<223>  anti-CS1 Luc63 light chain variable region

<400>  14
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Gly Ile Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Ser Tyr Pro Tyr
                85                  90                  95

Thr Phe Gly Gly Gly Thr Lys Leu Glu Ile Lys
            100                 105


<210>  15

<211>  120
<212>  PRT
<213>  artificial sequence

<220>
<223>  anti-CS1 Luc90 heavy chain variable region

<400>  15
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Ser Phe Thr Thr Tyr
            20                  25                  30

Trp Met Asn Trp Val Lys Gln Arg Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Arg Ser Thr Met Ile Ala Thr Arg Ala Met Asp Tyr Trp Gly Gln
            100                 105                 110

Gly Thr Ser Val Thr Val Ser Ser
        115                 120

<210>  16

<211>  107
<212>  PRT
<213>  artificial sequence

<220>
<223>  description of artificial sequence: synthetic oligopeptide

<220>
<223>  anti-CS1 Luc90 light chain variable region

<400>  16
Asp Ile Val Met Thr Gln Ser Gln Lys Ser Met Ser Thr Ser Val Gly
1               5                   10                  15

Asp Arg Val Ser Ile Thr Cys Lys Ala Ser Gln Asp Val Ile Thr Gly
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Asn Val Gln Ala
65                  70                  75                  80

Glu Asp Leu Ala Val Tyr Tyr Cys Gln Gln His Tyr Ser Thr Pro Leu
                85                  90                  95

Thr Phe Gly Ala Gly Thr Lys Leu Glu Leu Lys
            100                 105

<210>  17

<211>  121
<212>  PRT
<213>  artificial sequence

<220>
<223>  description of artificial sequence: synthetic oligopeptide

<220>
<223>  anti-CS1 Luc34 heavy chain variable region

<400>  17
Gln Val Gln Leu Gln Gln Ser Gly Ala Glu Leu Ala Arg Pro Gly Ala
1               5                   10                  15

Ser Val Lys Leu Ser Cys Lys Ala Ser Gly Tyr Thr Phe Thr Ser Tyr
            20                  25                  30

Trp Met Gln Trp Val Lys Gln Arg Pro Gly Gln Gly Leu Glu Trp Ile
        35                  40                  45

Gly Ala Ile Tyr Pro Gly Asp Gly Asp Thr Arg Tyr Thr Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Ala Ser Glu Asp Ser Ala Val Tyr Tyr Cys
                85                  90                  95

Ala Arg Gly Lys Val Tyr Tyr Gly Ser Asn Pro Phe Ala Tyr Trp Gly
            100                 105                 110

Gln Gly Thr Leu Val Thr Val Ser Ala
        115                 120


<210>  18

<211>  107
<212>  PRT
<213>  artificial sequence

<220>
<223>  description of artificial sequence: synthetic oligopeptide

<220>
<223>  anti-CS1 Luc34 light chain variable region

<400>  18
Asp Ile Gln Met Thr Gln Ser Ser Ser Tyr Leu Ser Val Ser Leu Gly
1               5                   10                  15

Gly Arg Val Thr Ile Thr Cys Lys Ala Ser Asp His Ile Asn Asn Trp
            20                  25                  30

Leu Ala Trp Tyr Gln Gln Lys Pro Gly Asn Ala Pro Arg Leu Leu Ile
        35                  40                  45

Ser Gly Ala Thr Ser Leu Glu Thr Gly Val Pro Ser Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Val Ala Thr Tyr Tyr Cys Gln Gln Tyr Trp Ser Thr Pro Trp
                85                  90                  95

Thr Phe Gly Gly Gly Thr Lys Leu Glu Ile Lys
            100                 105

<210>  19

<211>  120
<212>  PRT
<213>  artificial sequence

<220>
<223>  anti-CS1 LucX1 heavy chain variable region

<400>  19
Gln Val Gln Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Ser Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Lys Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Val Asp Ser Ala Val Tyr Phe Cys
                85                  90                  95

Ala Arg Ser Thr Met Ile Ala Thr Gly Ala Met Asp Tyr Trp Gly Gln
            100                 105                 110

Gly Thr Ser Val Thr Val Ser Ser
        115                 120

<210>  20

<211>  107
<212>  PRT
<213>  artificial sequence

<220>
<223>  description of artificial sequence: synthetic oligopeptide

<220>
<223>  anti-CS1 LucX1 light chain variable region

<400>  20
Glu Thr Thr Val Thr Gln Ser Pro Ala Ser Leu Ser Met Ala Ile Gly
1               5                   10                  15

Glu Lys Val Thr Ile Arg Cys Ile Thr Ser Thr Asp Ile Asp Asp Asp
            20                  25                  30

Met Asn Trp Tyr Gln Gln Lys Pro Gly Glu Pro Pro Lys Leu Leu Ile
        35                  40                  45

Ser Glu Gly Asn Thr Leu Arg Pro Gly Val Pro Ser Arg Phe Ser Ser
    50                  55                  60

Ser Gly Tyr Gly Thr Asp Phe Val Phe Thr Ile Glu Asn Met Leu Ser
65                  70                  75                  80

Glu Asp Val Ala Asp Tyr Tyr Cys Leu Gln Ser Asp Asn Leu Pro Leu
                85                  90                  95

Thr Phe Gly Gly Gly Thr Lys Leu Glu Ile Lys
            100                 105


<210>  21

<211>  120
<212>  PRT
<213>  artificial sequence

<220>
<223>  description of artificial sequence: synthetic oligopeptide

<220>
<223>  anti-CS1 LucX2 heavy chain variable region

<400>  21
Gln Val Gln Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Ser Ser
            20                  25                  30

Trp Met Asn Trp Val Lys Gln Arg Pro Gly Gln Gly Leu Glu Trp Ile

35                  40                  45

Gly Arg Ile Tyr Pro Gly Asp Gly Asp Thr Lys Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Val Asp Ser Ala Val Tyr Phe Cys
                85                  90                  95

Ala Arg Ser Thr Met Ile Ala Thr Gly Ala Met Asp Tyr Trp Gly Gln
            100                 105                 110

Gly Thr Ser Val Thr Val Ser Ser
        115                 120

<210>  22

<211>  108
<212>  PRT
<213>  artificial sequence

<220>
<223>  description of artificial sequence: synthetic oligopeptide

<220>
<223>  anti-CS1 LucX2 light chain variable region

<400>  22
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Thr Pro Pro
                85                  90                  95

Tyr Thr Phe Gly Gly Gly Thr Lys Leu Glu Ile Lys
            100                 105

## Description

### Field of the invention

The present invention relates to a new generation of chimeric antigen receptors (CAR or mcCAR) referred to as multi-chain CARs, which are made specific to the antigen CS1. Such CARs aim to redirect immune cell specificity and reactivity toward malignant cells expressing the tumor antigen CS1. The alpha, beta and gamma polypeptides composing these CARs are designed to assemble in juxtamembrane position, which forms flexible architecture closer to natural receptors, that confers optimal signal transduction. The invention encompasses the polynucleotides, vectors encoding said multi-chain CAR and the isolated cells expressing them at their surface, in particularly for their use in immunotherapy. The invention opens the way to efficient adoptive immunotherapy strategies for treating cancer, especially multiple myeloma.

### Background of the invention

Adoptive immunotherapy, which involves the transfer of autologous antigen-specific T cells generated *ex vivo*, is a promising strategy to treat viral infections and cancer. The T cells used for adoptive immunotherapy can be generated either by expansion of antigen-specific T cells or redirection of T cells through genetic engineering (Park, Rosenberg et al. 2011). Transfer of viral antigen specific T cells is a well-established procedure used for the treatment of transplant associated viral infections and rare viral-related malignancies. Similarly, isolation and transfer of tumor specific T cells has been shown to be successful in treating melanoma.

Novel specificities in T cells have been successfully generated through the genetic transfer of transgenic T cell receptors or chimeric antigen receptors (CARs) (Jena, Dotti et al. 2010). CARs are synthetic receptors consisting of a targeting moiety that is associated with one or more signaling domains to form a single-chain fusion molecule.

Single chain CARs have successfully allowed T cells to be redirected against antigens expressed at the surface of tumor cells from various malignancies including lymphomas and solid tumors (Jena, Dotti et al. 2010).

Multiple myeloma (MM) is a B-cell malignancy characterized by the aberrant clonal expansion of plasma cells (PCs) within the bone marrow, with an estimated 21,700 new cases and 10,710 deaths from MM identified in the United States in 2012 (Siegel R, et al. Cancer J Clin 2012 62:10-29). In 2013, it has been estimated that 22,350 individuals will be newly diagnosed with MM in the United States and 10,710 people will die from it, accounting for 20% of the deaths from all hematologic malignancies. Despite the use of proteasome inhibitors and immune-modulating drugs, which have improved overall survival (Palumbo A, et al. Leukemia 2009 23:449-456), MM remains an incurable malignancy (Podar K, et al. Leukemia 2009 23:10-24) for which novel therapeutic approaches are urgently needed.

The cell surface glycoprotein CS1 (also referred in the literature as SLAMF7, CD319 or CRACC - NCBI Reference Sequence: NP_067004.3) is highly and ubiquitously expressed on the surface of myeloma cells (Hsi ED, et al. Clin Cancer Res 2008 14:2775-84). CS1 is expressed at very low levels in the majority of immune effector cells, including natural killer (NK) cells, some subsets of T cells, and normal B cells, and is almost undetectable on myeloid cells (Hsi ED, et al. Clin Cancer Res 2008 14:2775-84). Notably, CS1 is negligibly expressed in human hematopoietic stem cells (Hsi ED, et al. Clin Cancer Res 2008 14:2775-84), which can be used for stem cell transplantation to treat hematologic malignancies, including MM. The functions of CS1 in MM remain incompletely understood, and it has been documented that CS1 may play a role in myeloma cell adhesion, clonogenic growth, and tumorigenicity (Benson DM Jr, et al. J Clin Oncol 2012 30:2013-5; Tai YT, et al. Blood 2009 113:4309-18).

In the context of developing therapeutic grade engineered immune cells that can target malignant or infected cells, the inventors have sought for improved CAR architectures, which would be closer to natural ones and likely to behave accordingly using any extracellular mono or multi-specific ligand binding domains. In WO2014039523, they described a new generation of CARs involving separate polypeptide sub-units according to the present invention, referred to as "multi-chain CARs". According to this architecture, the signaling domains and co-stimulatory domains are located on different polypeptide chains. Such multi-chain CARs can be derived from FcεRI, by replacing the high affinity IgE binding domain of FcεRI alpha chain by an extracellular ligand-binding domain such as scFv, whereas the N and/or C-termini tails of FcεRI beta and/or gamma chains are fused to signal transducing domains and co-stimulatory domains respectively. The extracellular ligand binding domain has the role of redirecting T-cell specificity towards cell targets, while the signal transducing domains activate the immune cell response. The fact that the different polypeptides derived from the alpha, beta and gamma polypeptides from FcεRI are transmembrane polypeptides sitting in juxtamembrane position, provides a more flexible architecture to CARs, improving specificity towards CS1 and reducing background activation of immune cells.

The inventors have now designed multi-chain CAR bearing scFv extracellular domain binding CS1, which are particularly suited to target malignant cells bearing CS1 as a marker. This was achieved, whereas so far very few antibodies had been so far described to act efficiently against CS1 positive cells for treating or preventing leukemia, in particular multiple myeloma.

### Description of the Figures:

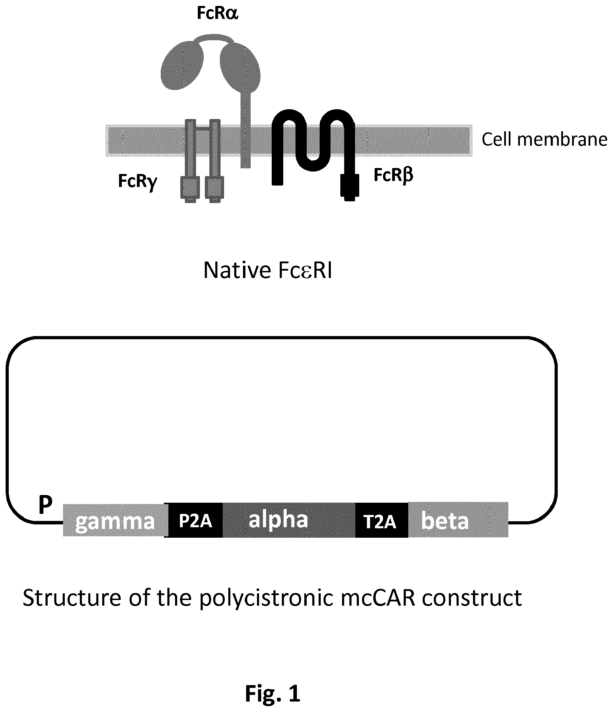

**Figure 1****:** Upper : Schematic representation of FcεRI from which derivate the multi-chain CAR architecture according to the invention. **Lower** General structure of the polycistronic construct encoding the CS1 muti-chain CAR according to the invention.

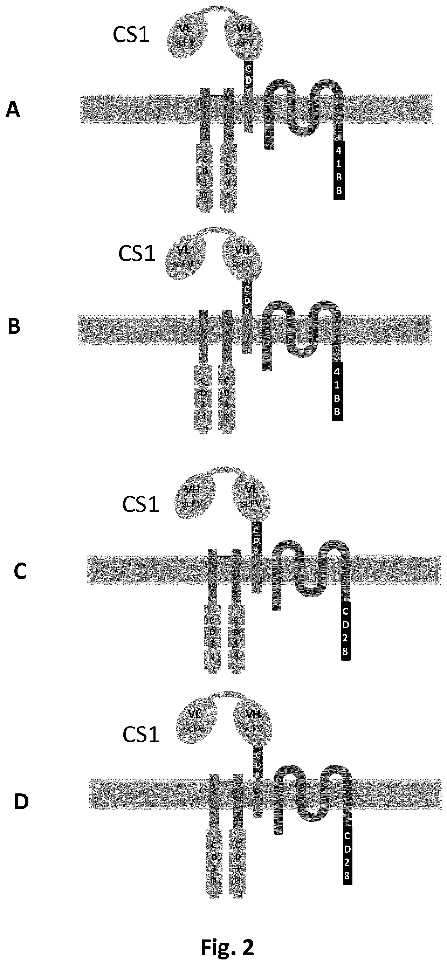

**Figure 2****:** Different architectures of the CS1 specific muti-chain CAR according to the invention. From left to right: polypeptide gamma (fused to ITAM of CD3zeta), polypeptide alpha (fused to ScFv), polypeptide beta (fused to co-stimulatory domain from either CD28 or 41BB). A and B: polypeptide beta is fused to co-stimulatory domain from 41BB, VL and VH fragments being in opposite orders. C and D: polypeptide beta is fused to co-stimulatory domain from CD28, VL and VH fragments being in opposite orders.

**Table 1: Exemplary sequences of the alpha polypeptide component of CS1 muti-chain CAR**

| **Functional domains** | **description** | **SEQ ID #** | **Raw amino acid sequence** |
|---|---|---|---|
| FcεRI α-SP | signal peptide | SEQ ID NO.1 | MAPAMESPTLLCVALLFFAPDGV LA |
| CD8α hinge | hinge | SEQ ID NO.2 | TTTPAPRPPTPAPTIASQPLSLRPE ACRPAAGGAVHTRGLDFACD |
| VH | | | See Table 5 |
| G4SX3Linker | Linker VH-VL | SEQ ID NO.3 | GGGGSGGGGSGGGGS |
| VL | | | See Table 5 |
| FcεRI α-TM-IC | Fc Receptor for IgE, alpha chain, transmembrane and intracellular domain | SEQ ID NO.4 | FFIPLLVVILFAVDTGLFISTQQQVT FLLKIKRTRKGFRLLNPHPKPNPKN N |

**Table 2: Exemplary sequences of the beta polypeptide component of CS1 muti-chain CAR**

| **Functional domains** | **description** | **SEQ ID #** | **Raw amino acid sequence** |
|---|---|---|---|
| FcεR1β-ΔITAM | Fc Receptor for IgE, beta chain, without ITAM | SEQ ID NO.5 | MDTESNRRANLALPQEPSSVPAF EVLEISPQEVSSGRLLKSASSPPLH TWLTVLKKEQEFLGVTQILTAMIC LCFGTVVCSVLDISHIEGDIFSSFKA GYPFWGAIFFSISGMLSIISERRNA TYLVRGSLGANTASSIAGGTGITILI INLKKSLAYIHIHSCQKFFETKCFM ASFSTEIVVMMLFLTILGLGSAVSL TICGAGEELKGNKVPE |
| 41BB-IC | 41BB co-stimulatory domain | SEQ ID NO.6 | KRGRKKLLYIFKQPFMRPVQTTQE EDGCSCRFPEEEEGGCEL |
| CD28-IC | CD28 co-stimulatory domain | SEQ ID NO.7 | RSKRSRGGHSDYMNMTPRRPGP TRKHYQPYAPPRDFAAYRS |

**Table 3: Exemplary sequences of the gamma polypeptide component of CS1 muti-chain CAR**

| **Functional domains** | **description** | **SEQ ID #** | **Raw amino acid sequence** |
|---|---|---|---|
| FcεRI γ-SP | signal peptide | SEQ ID NO.8 | MIPAVVLLLLLLVEQAAA |
| FcεRI γ - ΔITAM | Fc Receptor for IgE, gamma chain, without ITAM | SEQ ID NO.9 | LGEPQLCYILDAILFLYGIVLTLLYCR LKIQVRKAAITSYEKS |
| CD3ζ-IC | CD3zeta intracellular domain comprising ITAM | SEQ ID NO.10 | RVKFSRSADAPAYQQGQNQLYN ELNLGRREEYDVLDKRRGRDPEM GGKPRRKNPQEGLYNELQKDKM AEAYSEIGMKGERRRGKGHDGLY QGLSTATKDTYDALHMQALPPR |

**Table 4: skip peptides linking the polypeptides forming the mutli-subunit CAR**

| **Functional domains** | **description** | **SEQ ID #** | **Raw amino acid sequence** |
|---|---|---|---|
| GSG-P2A | GSG-P2A ribosomal skip peptide | SEQ ID NO.11 | GSGATNFSLLKQAGDVEENPGP |
| GSG-T2A | GSG-T2A ribosomal skip peptide | SEQ ID NO.12 | GSGEGRGSLLTCGDVEENPGP |

**Table 5: Sequence of exemplary CS1 binding regions**

| **CS1 ScFv sequences** | **SEQ ID #** | **Raw amino acid sequence** |
|---|---|---|
| Luc90 heavy chain variable region | SEQ ID NO.13 | QVQLQQPGAELVRPGASVKLSCKASGYSFTT YWMNWVKQRPGQGLEWIGMIHPSDSETRL NQKFKDKATLTVDKSSSTAYMQLSSPTSEDSA VYYCARSTMIATRAMDYWGQGTSVTVSS |
| Luc90 light chain variable region | SEQ ID NO.14 | DIVMTQSQKSMSTSVGDRVSITCKASQDVIT GVAWYQQKPGQSPKLLIYSASYRYTGVPDRF TGSGSGTDFTFTISNVQAEDLAVYYCQQHYST PLTFGAGTKLELK |
| Luc63 heavy chain variable region | SEQ ID NO.15 | EVKLLESGGGLVQPGGSLKLSCAASGFDFSRY WMSWVRQAPGKGLEWIGEINPDSSTINYTP SLKDKFIISRDNAKNTLYLQMSKVRSEDTALYY CARPDGNYWYFDVWGAGTTVTVSS |
| Luc63 light chain variable region | SEQ ID NO.16 | DIVMTQSHKFMSTSVGDRVSITCKASQDVGI AVAWYQQKPGQSPKLLIYWASTRHTGVPDR FTGSGSGTDFTLTISNVQSEDLADYFCQQYSS YPYTFGGGTKLEIK |
| Luc34 heavy chain variable region | SEQ ID NO.17 | QVQLQQSGAELARPGASVKLSCKASGYTFTS YWMQWVKQRPGQGLEWIGAIYPGDGDTR YTQKFKGKATLTADKSSSTAYMQLSSLASEDS AVYYCARGKVYYGSNPFAYWGQGTLVTVSA |
| Luc34 light chain variable region | SEQ ID NO.18 | DIQMTQSSSYLSVSLGGRVTITCKASDHINN WLAWYQQKPGNAPRLLISGATSLETGVPSRF SGSGSGKDYTLSITSLQTEDVATYYCQQYWST PWTFGGGTKLEIK |
| LucX1 heavy chain variable region | SEQ ID NO.19 | QVQLQQSGPELVKPGASVKISCKASGYAFSSS WMNWVKQRPGQGLEWIGRIYPGDGDTKY NGKFKGKATLTADKSSSTAYMQLSSLTSVDSA VYFCARSTMIATGAMDYWGQGTSVTVSS |
| LucX1 light chain variable region | SEQ ID NO.20 | ETTVTQSPASLSMAIGEKVTIRCITSTDIDDDM NWYQQKPGEPPKLLISEGNTLRPGVPSRFSSS GYGTDFVFTIENMLSEDVADYYCLQSDNLPLT FGGGTKLEIK |
| LucX2 heavy chain variable region | SEQ ID NO.21 | QVQLQQSGPELVKPGASVKISCKASGYAFSSS WMNWVKQRPGQGLEWIGRIYPGDGDTKY NGKFKGKATLTADKSSSTAYMQLSSLTSVDSA VYFCARSTMIATGAMDYWGQGTSVTVS |
| LucX2 light chain variable region | SEQ ID NO.22 | DIVMTQSHKFMSTSVGDRVSITCKASQDVST AVAWYQQKPGQSPKLLIYSASYRYTGVPDRF TGSGSGTDFTFTISSVQAEDLAVYYCQQHYST PPYTFGGGTKLEIK |

**Table 6: Exemplary Polypeptides forming CS1 muti-chain CAR**

| Multi chain CAR Designation | Precursor CS1 muti-chain CAR polypeptide structure | | | | | | | | | | | | |
|---|---|---|---|---|---|---|---|---|---|---|---|---|---|
| | Gamma polypeptide | | | | Alpha polypeptide | | | | | | | Beta polypeptide | |
| | FcεRI γ-SP | FcεRI γ-ΔITAM | CD3ζ-IC | P2A | FcεRI α-SP | CD8α hinge | VH | G4SX3 Linker | VL | FcεRI α-TM-IC | T2A | FcεR1β-ΔITAM | Co-stimulalion. domain |
| CS1-Luc63 (41BB) | SEQ ID NO.8 | SEQ ID NO.9 | SEQ ID NO.10 | SEQ ID NO.11 | SEQ ID NO.1 | SEQ ID NO.2 | SEQ ID NO.13 | SEQ ID NO.3 | SEQ ID NO.14 | SEQ ID NO.4 | SEQ ID NO.12 | SEQ ID NO.5 | SEQ ID NO.6 |
| CS1-Luc63 (CD28) | SEQ ID NO.8 | SEQ ID NO.9 | SEQ ID NO.10 | SEQ ID NO.11 | SEQ ID NO.1 | SEQ ID NO.2 | SEQ ID NO.13 | SEQ ID NO.3 | SEQ ID NO.14 | SEQ ID NO.4 | SEQ ID NO.12 | SEQ ID NO.5 | SEQ ID NO.7 |
| CS1-Luc90 (41BB) | SEQ ID NO.8 | SEQ ID NO.9 | SEQ ID NO.10 | SEQ ID NO.11 | SEQ ID NO.1 | SEQ ID NO.2 | SEQ ID NO.15 | SEQ ID NO.3 | SEQ ID NO.16 | SEQ ID NO.4 | SEQ ID NO.12 | SEQ ID NO.5 | SEQ ID NO.6 |
| CS1-Luc90 (CD28) | SEQ ID NO.8 | SEQ ID NO.9 | SEQ ID NO.10 | SEQ ID NO.11 | SEQ ID NO.1 | SEQ ID NO.2 | SEQ ID NO.15 | SEQ ID NO.3 | SEQ ID NO.16 | SEQ ID NO.4 | SEQ ID NO.12 | SEQ ID NO.5 | SEQ ID NO.7 |
| CS1-Luc34 (41BB) | SEQ ID NO.8 | SEQ ID NO.9 | SEQ ID NO.10 | SEQ ID NO.11 | SEQ ID NO.1 | SEQ ID NO.2 | SEQ ID NO.17 | SEQ ID NO.3 | SEQ ID NO.18 | SEQ ID NO.4 | SEQ ID NO.12 | SEQ ID NO.5 | SEQ ID NO.6 |
| CS1-Luc34 (41BB) | SEQ ID NO.8 | SEQ ID NO.9 | SEQ ID NO.10 | SEQ ID NO.11 | SEQ ID NO.1 | SEQ ID NO.2 | SEQ ID NO.17 | SEQ ID NO.3 | SEQ ID NO.18 | SEQ ID NO.4 | SEQ ID NO.12 | SEQ ID NO.5 | SEQ ID NO.7 |
| CS1-LucX1 (41BB) | SEQ ID NO.8 | SEQ ID NO.9 | SEQ ID NO.10 | SEQ ID NO.11 | SEQ ID NO.1 | SEQ ID NO.2 | SEQ ID NO.19 | SEQ ID NO.3 | SEQ ID NO.20 | SEQ ID NO.4 | SEQ ID NO.12 | SEQ ID NO.5 | SEQ ID NO.6 |
| CS1-LucX1 (CD28) | SEQ ID NO.8 | SEQ ID NO.9 | SEQ ID NO.10 | SEQ ID NO.11 | SEQ ID NO.1 | SEQ ID NO.2 | SEQ ID NO.19 | SEQ ID NO.3 | SEQ ID NO.20 | SEQ ID NO.4 | SEQ ID NO.12 | SEQ ID NO.5 | SEQ ID NO.7 |
| CS1-LucX2 (41BB) | SEQ ID NO.8 | SEQ ID NO.9 | SEQ ID NO.10 | SEQ ID NO.11 | SEQ ID NO.1 | SEQ ID NO.2 | SEQ ID NO.21 | SEQ ID NO.3 | SEQ ID NO.22 | SEQ ID NO.4 | SEQ ID NO.12 | SEQ ID NO.5 | SEQ ID NO.6 |
| CS1-LucX2 (CD28) | SEQ ID NO.8 | SEQ ID NO.9 | SEQ ID NO.10 | SEQ ID NO.11 | SEQ ID NO.1 | SEQ ID NO.2 | SEQ ID NO.21 | SEQ ID NO.3 | SEQ ID NO.22 | SEQ ID NO.4 | SEQ ID NO.12 | SEQ ID NO.5 | SEQ ID NO.7 |

### Detailed description of the invention

The present invention provides a CS1 specific multi-chain Chimeric Antigen Receptor (mc CAR) comprising:
- A transmembrane polypeptide from the alpha chain of high-affinity IgE receptor (FcεRI) fused to an extracellular CS1 ligand binding domain;
- A second transmembrane polypeptide from the gamma or beta chain of FcεRI fused to a signal transducing domain;
- A third transmembrane polypeptide from the gamma or beta chain of FcεRI comprising a co-stimulatory domain.

The present invention provides a CS1 specific multi-chain Chimeric Antigen Receptor as above, wherein said CS1 ligand binding domain fused to said alpha chain of FcεRI is a single-chain variable fragment (scFv) comprising heavy (V_{H}) and light (V_{L}) chains conferring specificity to CS1.

The present invention provides a CS1 specific multi-chain Chimeric Antigen Receptor as above, wherein said CS1 ligand binding domain fused to said alpha chain of FcεRI is a humanized single-chain variable fragment (scFv) comprising heavy (V_{H}) and light (V_{L}) chains conferring specificity to CS1.

The present invention provides a CS1 specific multi-chain Chimeric Antigen Receptor as above, wherein said V_{H} comprises a polypeptide sequence displaying at least 80%, at least 81%, at least 82% at least 83% at least 84% at least 85% at least 86% at least 87% at least 88% at least 89% at least 90% at least 91% at least 92% at least 93% at least 94% at least 95% at least 96% at least 97% at least 98% at least 99% or 100 % identity to one selected from SEQ ID NO. 13, SEQ ID NO. 15, SEQ ID NO. 17, SEQ ID NO. 19 and SEQ ID NO. 21.

The present invention provides a CS1 specific multi-chain Chimeric Antigen Receptor as above, wherein said V_{L} comprises a polypeptide displaying at least 80%, at least 81%, at least 82% at least 83% at least 84% at least 85% at least 86% at least 87% at least 88% at least 89% at least 90% at least 91% at least 92% at least 93% at least 94% at least 95% at least 96% at least 97% at least 98% at least 99% or 100 % identity to one selected from SEQ ID NO. 14, SEQ ID NO. 16, SEQ ID NO. 18, SEQ ID NO. 20 and SEQ ID NO. 22.

The present invention provides a CS1 specific multi-chain Chimeric Antigen Receptor as above, wherein said alpha chain of FcεRI is fused to said extracellular ligand-binding domain by a hinge from CD8α, IgG1 or FcRIIIα proteins.

The present invention provides a CS1 specific multi-chain Chimeric Antigen Receptor as above, wherein said hinge comprises a polypeptide sequence displaying % at least 90% at least 91% at least 92% at least 93% at least 94% at least 95% at least 96% at least 97% at least 98% at least 99% or 100 % identity to SEQ ID NO.2.

The present invention provides a CS1 specific multi-chain Chimeric Antigen Receptor as above, wherein said signal transducing domain fused to the gamma or beta chain of FcεRI is from the TCR zeta chain, the FCεRβ chain, the FcεRIγ chain, or includes an immunoreceptor tyrosine-based activation motif (ITAM).

In a preferred embodiment, the present invention provides a CS1-L specific multi-chain Chimeric Antigen Receptor (mc CAR) comprising:
- A transmembrane polypeptide from the alpha chain of high-affinity IgE receptor (FcεRI) fused to an extracellular CS1-S ligand binding domain;
- A second transmembrane polypeptide from the gamma or beta chain of FcεRI fused to a signal transducing domain;
- A third transmembrane polypeptide from the gamma or beta chain of FcεRI comprising a co-stimulatory domain.
The present invention provides a CS1 specific multi-chain Chimeric Antigen Receptor as above, wherein said signal transducing domain is from CD3zeta.

In a preferred embodiment, conservative sequence modifications are introduced into an antibody, into an antibody fragment or in any of the other parts of the CAR molecule of the invention by standard techniques known in the art, such as site-directed mutagenesis, PCR-mediated mutagenesis or by employing optimized germline sequences.

As used herein, the term "conservative sequence modifications" or "humanization" is intended to refer to amino acid modifications that do not significantly affect or alter the binding characteristics of the CAR and/or that do not significantly affect the activity of the CAR containing the modified amino acid sequence and reduce or abolish a human antimouse antibody (HAMA) response. Such conservative modifications include amino acid substitutions, additions and deletions in said antibody fragment in said CAR and/or any of the other parts of said CAR molecule.

Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR of the invention can be replaced with other amino acid residues from the same side chain family and the altered CAR can be tested for the ability to bind CS1 using the functional assays described previously in PCT/EP2015/055848 incorporated herein by reference and adaptated for CS1 .

An anti-CS1 msCAR of the invention is provided by engineering an antibody specific for CS-1 in particular an antibody specific for human CS1 and more particularly by engineering an antibody specific for human CS1 that do not induce HAMA response when expressed in a human T cell.

The present invention provides a CS1 specific multi-chain Chimeric Antigen Receptor as above wherein said signal transducing domain comprises a polypeptide sequence displaying % at least 90% at least 91% at least 92% at least 93% at least 94% at least 95% at least 96% at least 97% at least 98% at least 99% or 100 % identity to SEQ ID NO.10.

The present invention provides a CS1 specific multi-chain Chimeric Antigen Receptor as above, wherein said second or third polypeptide comprises a co-stimulatory domain from the cytoplasmic domain of a costimulatory molecule selected from CD27, CD28, 4-1BB, OX40, CD30, CD40, PD-1, ICOS, lymphocyte function-associated antigen-1 (LFA-1), CD2, CD7, CD8, LIGHT, NKG2C, B7-H3, a ligand that specifically binds with CD83, and any combination thereof.

The present invention provides a CS1 specific multi-chain Chimeric Antigen Receptor as above, wherein said co-stimulatory domain is from 4-1BB and comprises a polypeptide sequence displaying at least 90 % identity to SEQ ID NO.6.

The present invention provides a CS1 specific multi-chain Chimeric Antigen Receptor as above, wherein said co-stimulatory domain is from CD28 and comprises a polypeptide sequence displaying % at least 90% at least 91% at least 92% at least 93% at least 94% at least 95% at least 96% at least 97% at least 98% at least 99% or 100 % identity to SEQ ID NO.7.

The present invention provides a polypeptide encoding a CS1 specific multi-chain Chimeric Antigen Receptor as above, comprising a polypeptide sequence displaying at least 80 % identity to the full amino acid sequence of CS1-Luc63, CS1-Luc90, CS1-Luc34 or CS1-Luc63, as referred to in Table 6.

In a preferred embodiment, the present invention provides a polypeptide encoding a CS1 specific multi-chain Chimeric Antigen Receptor as above, comprising a polypeptide sequence displaying at least 80 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 81 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 82 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 83 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 84 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 85 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 86 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 87 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 88 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 89 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 90 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 91 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 92 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 93 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 94 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 95 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 96 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 97 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 98 % identity to the full amino acid sequence of CS1-Luc63.

In a preferred embodiment, the present invention provides a polypeptide encoding a CS1 specific multi-chain Chimeric Antigen Receptor as above, comprising a polypeptide sequence displaying at least 99 % identity to the full amino acid sequence of CS1-Luc63.

The present invention provides a polynucleotide comprising a nucleic acid sequence encoding a CS1 specific multi-chain Chimeric Antigen Receptor as above.

The present invention provides a vector comprising a polynucleotide as above.

The present invention provides a method of engineering an immune cell comprising:
(a) Providing an immune cell;
(b) Expressing at the surface of said cells at least one multi-chain Chimeric Antigen Receptor as above.

The present invention provides a method of engineering an immune cell as above comprising:
(a) Providing an immune cell;
(b) Introducing into said cell at least one polynucleotide encoding polypeptides composing at least one multi-chain Chimeric Antigen Receptor according to any one the above;
(c) Expressing said polynucleotides into said cell.

The present invention provides a method of engineering an immune cell as above comprising:
(a) Providing an immune cell;
(b) Expressing at the surface of said cell a population of multi-chain Chimeric Antigen Receptors as above each one comprising different extracellular ligand-binding domains.

The present invention provides a method of engineering an immune cell of as above comprising:
(a) Providing an immune cell;
(b) Introducing into said cell at least one polynucleotide encoding polypeptides composing a population of multi-chain Chimeric Antigen Receptors according to any one of the above each one comprising different extracellular ligand binding domains.
(c) Expressing said polynucleotides into said cell.

The present invention provides an isolated immune cell obtainable from the method according to any one of the above.

The present invention provides an isolated immune cell comprising at least one multi-chain Chimeric Antigen Receptor according to any one of the above.

The present invention provides an isolated immune cell as above for its use as a medicament.

The present invention provides an isolated cell according to the above derived from, NK cells, inflammatory T-lymphocytes, cytotoxic T-lymphocytes, regulatory T-lymphocytes or helper T-lymphocytes.

The present invention provides an isolated immune cell derived from, NK cells, inflammatory T-lymphocytes, cytotoxic T-lymphocytes, regulatory T-lymphocytes or helper T-lymphocytes endowed with an anti-CS1 CAR for its use as a medicament.

Unless specifically defined herein, all technical and scientific terms used have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

### Multi-chain Chimeric Antigen Receptor (CAR)

The present invention relates to a multi-chain chimeric antigen receptor (CAR) particularly adapted to immune cells used in immunotherapy.
The multi-chain CAR according to the invention generally comprises at least:
- one transmembrane polypeptide comprising at least one extracellular ligand-biding domain and;
- one transmembrane polypeptide comprising at least one signal-transducing domain;
   such that said polypeptides assemble together to form a multi-chain Chimeric Antigen Receptor.
The term "extracellular ligand-binding domain" as used herein is defined as an oligo- or polypeptide that is capable of binding a ligand. Preferably, the domain will be capable of interacting with a cell surface molecule.

In a preferred embodiment, said extracellular ligand-binding domain is a single chain antibody fragment (scFv) comprising the light (*V_{L}*) and the heavy (*V_{H}*) variable fragment of a target antigen specific monoclonal antibody specific to CS1 joined by a flexible linker. In a preferred embodiment, said scFv is an anti-CS1 scFV, preferably provided in Table 5 as SEQ ID NO.13 to 22. Binding domain specific to CS1 other than scFv can also be used for predefined targeting of lymphocytes, such as camelid or shark (VNAR) single-domain antibody fragments or receptor ligands like a vascular endothelial growth factor polypeptide, an integrin-binding peptide, heregulin or an IL-13 mutein, antibody binding domains, antibody hypervariable loops or CDRs as non-limiting examples.

In a preferred embodiment said first transmembrane polypeptide further comprises a stalk region between said extracellular ligand-binding domain and said transmembrane domain. The term "stalk region" used herein generally means any oligo- or polypeptide that functions to link the transmembrane domain to the extracellular ligand-binding domain. In particular, stalk region are used to provide more flexibility and accessibility for the extracellular ligand-binding domain. A stalk region may comprise up to 300 amino acids, preferably 10 to 100 amino acids and most preferably 25 to 50 amino acids. Stalk region may be derived from all or part of naturally occurring molecules, such as from all or part of the extracellular region of CD8, CD4 or CD28, or from all or part of an antibody constant region. Alternatively the stalk region may be a synthetic sequence that corresponds to a naturally occurring stalk sequence, or may be an entirely synthetic stalk sequence. In a preferred embodiment said stalk region is a part of human CD8 alpha chain (e.g. NP_001139345.1) (SEQ ID NO: 2).

Thus, the expression of multi-chain CAR in immune cells results in modified cells that selectively and eliminate defined targets, including but not limited to malignant cells carrying a respective tumor-associated surface antigen or virus infected cells carrying a virus-specific surface antigen, or target cells carrying a lineage-specific or tissue-specific surface antigen.

Downregulation or mutation of target antigens is commonly observed in cancer cells, creating antigen-loss escape variants. Thus, to offset tumor escape and render immune cell more specific to target, the multi-chain CAR can comprise several extracellular ligand-binding domains, to simultaneously bind different elements in target thereby augmenting immune cell activation and function. In one embodiment, the extracellular ligand-binding domains can be placed in tandem on the same transmembrane polypeptide, and optionally can be separated by a linker. In another embodiment, said different extracellular ligand-binding domains can be placed on different transmembrane polypeptides composing the multi-chain CAR. In another embodiment, the present invention relates to a population of multi-chain CARs comprising each one different extracellular ligand binding domains. In a particular, the present invention relates to a method of engineering immune cells comprising providing an immune cell and expressing at the surface of said cell a population of multi-chain CAR each one comprising different extracellular ligand binding domains. In another particular embodiment, the present invention relates to a method of engineering an immune cell comprising providing an immune cell and introducing into said cell polynucleotides encoding polypeptides composing a population of multi-chain CAR each one comprising different extracellular ligand binding domains. In a particular embodiment the method of engineering an immune cell comprises expressing at the surface of the cell at least a part of FcεRI beta and/or gamma chain fused to a signal-transducing domain and several part of FcεRI alpha chains fused to different extracellular ligand binding domains. In a more particular embodiment, said method comprises introducing into said cell at least one polynucleotide which encodes a part of FcεRI beta and/or gamma chain fused to a signal-transducing domain and several FcεRI alpha chains fused to different extracellular ligand biniding domains. By population of multi-chain CARs, it is meant at least two, three, four, five, six or more multi-chain CARs each one comprising different extracellular ligand binding domains. The different extracellular ligand binding domains according to the present invention can preferably simultaneously bind different elements in target thereby augmenting immune cell activation and function.

The present invention also relates to an isolated immune cell which comprises a population of multi-chain CARs each one comprising different extracellular ligand binding domains.

The signal transducing domain or intracellular signaling domain of the multi-chain CAR of the invention is responsible for intracellular signaling following the binding of extracellular ligand binding domain to the target resulting in the activation of the immune cell and immune response. In other words, the signal transducing domain is responsible for the activation of at least one of the normal effector functions of the immune cell in which the multi-chain CAR is expressed. For example, the effector function of a T cell can be a cytolytic activity or helper activity including the secretion of cytokines. Thus, the term "signal tansducing domain" refers to the portion of a protein which transduces the effector signal function signal and directs the cell to perform a specialized function.

Preferred examples of signal transducing domain for use in multi-chain CAR can be the cytoplasmic sequences of the Fc receptor or T cell receptor and co-receptors that act in concert to initiate signal transduction following antigen receptor engagement, as well as any derivate or variant of these sequences and any synthetic sequence that as the same functional capability. Signal transduction domain comprises two distinct classes of cytoplasmic signaling sequence, those that initiate antigen-dependent primary activation, and those that act in an antigen-independent manner to provide a secondary or co-stimulatory signal. Primary cytoplasmic signaling sequence can comprise signaling motifs which are known as immunoreceptor tyrosine-based activation motifs of ITAMs. ITAMs are well defined signaling motifs found in the intracytoplasmic tail of a variety of receptors that serve as binding sites for syk/zap70 class tyrosine kinases. Examples of ITAM used in the invention can include as non limiting examples those derived from TCRzeta, FcRgamma, FcRbeta, FcRepsilon, CD3gamma, CD3delta, CD3epsilon, CD5, CD22, CD79a, CD79b and CD66d. In a preferred embodiment, the signaling transducing domain of the multi-chain CAR can comprise the CD3zeta signaling domain, or the intracytoplasmic domain of the FcεRI beta or gamma chains.

In particular embodiment the signal transduction domain of the multi-chain CAR of the present invention comprises a co-stimulatory signal molecule. A co-stimulatory molecule is a cell surface molecule other than an antigen receptor or their ligands that is required for an efficient immune response.

"Co-stimulatory ligand" refers to a molecule on an antigen presenting cell that specifically binds a cognate co-stimulatory molecule on a T-cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A co-stimulatory ligand can include but is not limited to CD7, B7-1 (CD80), B7-2 (CD86), PD-L1, PD-L2, 4-1BBL, OX40L, inducible costimulatory igand (ICOS-L), intercellular adhesion molecule (ICAM, CD30L, CD40, CD70, CD83, HLA-G, MICA, M1CB, HVEM, lymphotoxin beta receptor, 3/TR6, ILT3, ILT4, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as but not limited to, CD27, CD28, 4-IBB, OX40, CD30, CD40, PD-1, ICOS, lymphocyte function-associated antigen-1 (LFA-1), CD2, CD7, LTGHT, NKG2C, B7-H3, a ligand that specifically binds with CD83.

A "co-stimulatory molecule" refers to the cognate binding partner on a T-cell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor. Examples of costimulatory molecules include CD27, CD28, CD8, 4-1BB (CD137), OX40, CD30, CD40, PD-1, ICOS, lymphocyte function-associated antigen-1 (LFA-1), CD2, CD7, LIGHT, NKG2C, B7-H3 and a ligand that specifically binds with CD83 and the like.

In another particular embodiment, said signal transducing domain is a TNFR-associated Factor 2 (TRAF2) binding motifs, intracytoplasmic tail of costimulatory TNFR member family. Cytoplasmic tail of costimulatory TNFR family member contains TRAF2 binding motifs consisting of the major conserved motif (P/S/A)X(Q/E)E) or the minor motif (PXQXXD), wherein X is any amino acid. TRAF proteins are recruited to the intracellular tails of many TNFRs in response to receptor trimerization.

In a preferred embodiment, the signal transduction domain of the multi-chain CAR of the present invention comprises a part of co-stimulatory signal molecule selected from the group consisting of 4-1BB (GenBank: AAA53133.) and CD28 (NP_006130.1).

The distinguishing features of appropriate transmembrane polypeptides comprise the ability to be expressed at the surface of an immune cell, in particular lymphocyte cells or Natural killer (NK) cells, and to interact together for directing cellular response of immune cell against a predefined target cell. The different transmembrane polypeptides of the multi-chain CAR of the present invention comprising an extracellular ligand-biding domain and/or a signal transducing domain interact together to take part in signal transduction following the binding with a target ligand and induce an immune response. The transmembrane domain can be derived either from a natural or from a synthetic source. The transmembrane domain can be derived from any membrane-bound or transmembrane protein. As non limiting examples, the transmembrane polypeptide can be a subunit of the T cell receptor such as α, β, γ or δ, polypeptide constituting CD3 complex, IL2 receptor p55 (α chain), p75 (β chain) or γ chain, subunit chain of Fc receptors, in particular Fcγ receptor III or CD proteins. Alternatively the transmembrane domain can be synthetic and can comprise predominantly hydrophobic residues such as leucine and valine.

The term "derived from" means a polypeptide having an amino acid sequence which is equivalent to that an Fcε receptor which include one or more amino acid modification(s) of the sequence of the Fcε receptor. Such amino acid modification(s) may include amino acid substitution(s), deletion(s), addition(s) or a combination of any of those modifications, and may alter the biological activity of the Fc binding region relative to that of an Fc receptor. On the other hand, Fc binding regions derived from a particular Fc receptor may include one or more amino acid modification(s) which do not substantially alter the biological activity of the Fc binding region relative to that of an Fc receptor. Amino acid modification(s) of this kind will typically comprise conservative amino acid substitution(s).

In a particular embodiment, the multi-chain CAR comprises a transmembrane polypeptide derived from a FcεRI chain. In more particular embodiment FcεRI chain is a FcεRI α chain, in which the extracellular domain is replaced by an extracellular ligand-binding domain, preferably by a scFV directed against CS1.

In more particular embodiment, said multi-chain CAR can comprise a part of FcεRI alpha chain and a part of FcεRI beta chain or variant thereof such that said FcεRI chains spontaneously dimerize together to form a dimeric Chimeric Antigen Receptor. In another embodiment, the multi-chain Chimeric Antigen can comprise a part of FcεRI alpha chain and a part of a FcεRI gamma chain or variant thereof such that said FcεRI chains spontaneously trimerize together to form a trimeric Chimeric Antigen Receptor, and in another embodiment the multi-chain Chimeric Antigen Receptor can comprise a part of FcεRI alpha chain, a part of FcεRI beta chain and a part of FcεRI gamma chain or variants thereof such that said FcεRI chains spontaneously tetramerize together to form a tetrameric Chimeric Antigen Receptor.

As non limiting example, different versions of multi-chain CAR are illustrated in Figure 3. In a more preferred embodiment, the multi-chain CARs of the present invention comprises a polypeptide with amino acid sequence as set forth in Table 6. In a preferred embodiment the multi-chain CAR comprise a polypeptide with amino acid sequence that has at least 70%, preferably at least 80%, more preferably at least 90 %, 95 % 97 % or 99 % sequence identity with such amino acid sequences.

In a more preferred embodiment the multi-chain CAR comprise a polypeptide with amino acid sequence that has at least 70%, preferably at least 80%, more preferably at least 90 %, 95 % 97 % or 99 % sequence identity comprising an amino acid sequence SEQ ID NO:15 and/or SEQ ID NO:16.
"identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means, in particular by reverse translating its amino acid sequence using the genetic code.

### Polynucleotides, vectors:

The present invention also relates to polynucleotides, vectors encoding the above described multi-chain CAR according to the invention. The present invention provides polynucleotides, including DNA and RNA molecules that encode the transmembrane polypeptides disclosed herein that can be included in the multi-chain CAR. In particular, the invention relates to a polynucleotide comprising a nucleic acid sequence encoding at least one transmembrane polypeptide composing the multi-chain CAR as described above. More particularly the invention relates to a polynucleotide comprising two or more nucleic acid sequences encoding transmembrane polypeptides composing the multi-chain CAR as described above.

The polynucleotide may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell).

In a particular embodiment, the different nucleic acid sequences can be included in one polynucleotide or vector which comprises a nucleic acid sequence encoding ribosomal skip sequence such as a sequence encoding a 2A peptide. 2A peptides, which were identified in the Aphthovirus subgroup of picornaviruses, causes a ribosomal "skip" from one codon to the next without the formation of a peptide bond between the two amino acids encoded by the codons (see Donnelly et al., J. of General Virology 82: 1013-1025 (2001); Donnelly et al., J. of Gen. Virology 78: 13-21 (1997); Doronina et al., Mol. And. Cell. Biology 28(13): 4227-4239 (2008); Atkins et al., RNA 13: 803-810 (2007)). By "codon" is meant three nucleotides on an mRNA (or on the sense strand of a DNA molecule) that are translated by a ribosome into one amino acid residue. Thus, two polypeptides can be synthesized from a single, contiguous open reading frame within an mRNA when the polypeptides are separated by a 2A oligopeptide sequence that is in frame. Such ribosomal skip mechanisms are well known in the art and are known to be used by several vectors for the expression of several proteins encoded by a single messenger RNA. As non-limiting example, in the present invention, 2A peptides have been used to express into the cell the different polypeptides of the multi-chain CAR.

To direct, transmembrane polypeptide such as FcεR into the secretory pathway of a host cell, a secretory signal sequence (also known as a leader sequence, prepro sequence or pre sequence) is provided in polynucleotide sequence or vector sequence. The secretory signal sequence may be that of FcεR, or may be derived from another secreted protein (e.g., t-PA) or synthesized *de novo.* The secretory signal sequence is operably linked to the transmembrane nucleic acid sequence, i.e., the two sequences are joined in the correct reading frame and positioned to direct the newly synthesized polypeptide into the secretory pathway of the host cell. Secretory signal sequences are commonly positioned 5' to the nucleic acid sequence encoding the polypeptide of interest, although certain secretory signal sequences may be positioned elsewhere in the nucleic acid sequence of interest (see, e.g., Welch et al., U.S. Patent No. 5,037,743; Holland et al., U.S. Patent No. 5,143,830). In a preferred embodiment the signal peptide comprises the residues 1 to 25 of the FcεRI alpha chain (NP_001992.1) and has the amino acid sequence SEQ ID NO: 205.

Those skilled in the art will recognize that, in view of the degeneracy of the genetic code, considerable sequence variation is possible among these polynucleotide molecules. Preferably, the nucleic acid sequences of the present invention are codon-optimized for expression in mammalian cells, preferably for expression in human cells. Codon-optimization refers to the exchange in a sequence of interest of codons that are generally rare in highly expressed genes of a given species by codons that are generally frequent in highly expressed genes of such species, such codons encoding the amino acids as the codons that are being exchanged.

### Methods of engineering an immune cell:

In encompassed particular embodiment, the invention relates to a method of preparing immune cells for immunotherapy comprising introducing into said immune cells the polypeptides composing said multi-chain CAR and expanding said cells. In particular embodiment, the invention relates to a method of engineering an immune cell comprising providing a cell and expressing at the surface of said cell at least one multi-chain CAR as described above. In particular embodiment, the method comprises transforming the cell with at least one polynucleotide encoding polypeptides composing at least one multi-chain CAR as described above, and expressing said polynucleotides into said cell.

In another embodiment, the present invention relates to a method of preparing cells for immunotherapy comprising introducing into said cells the different polypeptides composing said multi-chain CAR and expanding said cells. In a preferred embodiment, said polynucleotides are included in lentiviral vectors in view of being stably expressed in the cells.

### Delivery methods

The different methods described above involve introducing multi-chain CAR, pTalpha or functional variants thereof, rare cutting endonuclease, TALE-nuclease, CAR optionally with DNA-end processing enzyme or exogenous nucleic acid into a cell.

As non-limiting example, said multi-chain CAR can be introduced as transgenes encoded by one or as different plasmidic vectors. Different transgenes can be included in one vector which comprises a nucleic acid sequence encoding ribosomal skip sequence such as a sequence encoding a 2A peptide. 2A peptides, which were identified in the Aphthovirus subgroup of picornaviruses, causes a ribosomal "skip" from one codon to the next without the formation of a peptide bond between the two amino acids encoded by the codons (see Donnelly et al., J. of General Virology 82: 1013-1025 (2001); Donnelly et al., J. of Gen. Virology 78: 13-21 (1997); Doronina et al., Mol. And. Cell. Biology 28(13): 4227-4239 (2008); Atkins et al., RNA 13: 803-810 (2007)). By "codon" is meant three nucleotides on an mRNA (or on the sense strand of a DNA molecule) that are translated by a ribosome into one amino acid residue. Thus, two polypeptides can be synthesized from a single, contiguous open reading frame within an mRNA when the polypeptides are separated by a 2A oligopeptide sequence that is in frame. Such ribosomal skip mechanisms are well known in the art and are known to be used by several vectors for the expression of several proteins encoded by a single messenger RNA. As non-limiting example, in the present invention, 2A peptides have been used to express into the cell the rare-cutting endonuclease and a DNA end-processing enzyme or the different polypeptides of the multi-chain CAR.

Said plasmid vector can also contain a selection marker which provides for identification and/or selection of cells which received said vector.

Polypeptides may be synthesized *in situ* in the cell as a result of the introduction of polynucleotides encoding said polypeptides into the cell. Alternatively, said polypeptides could be produced outside the cell and then introduced thereto. Methods for introducing a polynucleotide construct into animal cells are known in the art and including as non limiting examples stable transformation methods wherein the polynucleotide construct is integrated into the genome of the cell, transient transformation methods wherein the polynucleotide construct is not integrated into the genome of the cell and virus mediated methods. Said polynucleotides may be introduced into a cell by for example, recombinant viral vectors (e.g. retroviruses, adenoviruses), liposome and the like. For example, transient transformation methods include for example microinjection, electroporation or particle bombardment. Said polynucleotides may be included in vectors, more particularly plasmids or virus, in view of being expressed in cells.

### - Electroporation

In particular embodiment of the invention, polynucleotides encoding polypeptides according to the present invention can be mRNA which is introduced directly into the cells, for example by electroporation. The inventors determined the optimal condition for mRNA electroporation in T-cell.

The inventor used the cytoPulse technology which allows, by the use of pulsed electric fields, to transiently permeabilize living cells for delivery of material into the cells. The technology, based on the use of PulseAgile (Cellectis property) electroporation waveforms grants the precise control of pulse duration, intensity as well as the interval between pulses (U.S. patent 6,010,613 and International PCT application WO2004083379). All these parameters can be modified in order to reach the best conditions for high transfection efficiency with minimal mortality. Basically, the first high electric field pulses allow pore formation, while subsequent lower electric field pulses allow to move the polynucleotide into the cell. In one aspect of the present invention, the inventor describe the steps that led to achievement of >95% transfection efficiency of mRNA in T cells, and the use of the electroporation protocol to transiently express different kind of proteins in T cells. In particular the invention relates to a method of transforming T cell comprising contacting said T cell with RNA and applying to T cell an agile pulse sequence consisting of:
(a) one electrical pulse with a voltage range from 2250 to 3000 V per centimeter, a pulse width of 0.1 ms and a pulse interval of 0.2 to 10 ms between the electrical pulses of step (a) and (b);
(b) one electrical pulse with a voltage range from 2250 to 3000 V with a pulse width of 100 ms and a pulse interval of 100 ms between the electrical pulse of step (b) and the first electrical pulse of step (c) ; and
(c) 4 electrical pulses with a voltage of 325 V with a pulse width of 0.2 ms and a pulse interval of 2 ms between each of 4 electrical pulses.
In particular embodiment, the method of transforming T cell comprising contacting said T cell with RNA and applying to T cell an agile pulse sequence consisting of:
(a) one electrical pulse with a voltage of 2250, 2300, 2350, 2400, 2450, 2500, 2550, 2400, 2450, 2500, 2600, 2700, 2800, 2900 or 3000V per centimeter, a pulse width of 0.1 ms and a pulse interval of 0.2, 0.5, 1, 2, 3, 4, 5, 6, 7, 8, 9 or 10 ms between the electrical pulses of step (a) and (b);
(b) one electrical pulse with a voltage range from 2250, of 2250, 2300, 2350, 2400, 2450, 2500, 2550, 2400, 2450, 2500, 2600, 2700, 2800, 2900 or 3000V with a pulse width of 100 ms and a pulse interval of 100 ms between the electrical pulse of step (b) and the first electrical pulse of step (c); and
(c) 4 electrical pulses with a voltage of 325 V with a pulse width of 0.2 ms and a pulse interval of 2 ms between each of 4 electrical pulses.

Any values included in the value range described above are disclosed in the present application. Electroporation medium can be any suitable medium known in the art. Preferably, the electroporation medium has conductivity in a range spanning 0.01 to 1.0 milliSiemens.

In particular embodiments, as non limiting examples, said RNA encodes a rare-cutting endonuclase, one monomer of the rare-cutting endonuclease such as Half-TALE-nuclease, a Chimeric Antigen Receptor, at least one component of the multi-chain chimeric antigen receptor, a pTalpha or functional variant thereof, an exogenous nucleic acid, one additional catalytic domain.

### Modified T-cells

The present invention also relates to isolated cells or cell lines susceptible to be obtained by said method to engineer cells. In particular said isolated cell comprises at least one multi-chain CAR as described above. In another embodiment, said isolated cell comprises a population of multi-chain CARs each one comprising different extracellular ligand binding domains. In particular, said isolated cell comprises exogenous polynucleotide sequences encoding polypeptides composing at least one multi-chain CAR of the invention.

In the scope of the present invention is also encompassed an isolated immune cell, preferably a T-cell obtained according to any one of the methods previously described. Said immune cell refers to a cell of hematopoietic origin functionally involved in the initiation and/or execution of innate and/or adaptative immune response. Said immune cell according to the present invention can be derived from a stem cell. The stem cells can be adult stem cells, embryonic stem cells, more particularly non-human stem cells, cord blood stem cells, progenitor cells, bone marrow stem cells, induced pluripotent stem cells, totipotent stem cells or hematopoietic stem cells. Representative human cells are CD34+ cells. Said isolated cell can also be a dendritic cell, killer dendritic cell, a mast cell, a NK-cell, a B-cell or a T-cell selected from the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. Prior to expansion and genetic modification of the cells of the invention, a source of cells can be obtained from a subject through a variety of non-limiting methods. Cells can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available and known to those skilled in the art, may be used. In another embodiment, said cell can be derived from a healthy donor, from a patient diagnosed with cancer or from a patient diagnosed with an infection. In another embodiment, said cell is part of a mixed population of cells which present different phenotypic characteristics. In the scope of the present invention is also encompassed a cell line obtained from a transformed T- cell according to the method previously described. Modified cells resistant to an immunosuppressive treatment and susceptible to be obtained by the previous method are encompassed in the scope of the present invention.

As mentioned previously, such cells can be also genetically engineered to inactivate one or several genes selected, for instance, from the group consisting of CD52, GR, TCR alpha, TCR beta, HLA gene, immune check point genes such as PD1 and CTLA-4, or can express a pTalpha transgene.

In another embodiment, TCR is rendered not functional in the cells according to the invention by inactivating TCR alpha gene and/or TCR beta gene(s). The above strategies are used more particularly to avoid GvHD. In a particular aspect of the present invention is a method to obtain modified cells derived from an individual, wherein said cells can proliferate independently of the Major Histocompatibility Complex signaling pathway. Said method comprises the following steps:
(a) Recovering cells from said individual;
(b) Genetically modifying said cells ex-vivo by inactivating TCR alpha or TCR beta genes;
(c) Cultivating genetically modified T-cells in vitro in appropriate conditions to amplify said cells.
Modified cells, which can proliferate independently of the Major Histocompatibility Complex signaling pathway, susceptible to be obtained by this method are encompassed in the scope of the present invention. Said modified cells can be used in a particular aspect of the invention for treating patients in need thereof against Host versus Graft (HvG) rejection and Graft versus Host Disease (GvHD); therefore in the scope of the present invention is a method of treating patients in need thereof against Host versus Graft (HvG) rejection and Graft versus Host Disease (GvHD) comprising treating said patient by administering to said patient an effective amount of modified cells comprising inactivated TCR alpha and/or TCR beta genes.

In a more preferred embodiment, said method comprises:
(a) Providing a T-cell, preferably from a cell culture or from a blood sample;
(b) Transforming said T cell with nucleic acid encoding a rare-cutting endonuclease able to selectively inactivate by DNA cleavage, preferably by double-strand break at least one gene encoding a component of the T-cell receptor (TCR);
(c) Expressing said rare-cutting endonucleases into said T-cells;
(d) Sorting the transformed T-cells, which do not express TCR on their cell surface;
(e) Expanding said cells.
In another embodiment, said rare-cutting endonuclease can be a meganuclease, a Zinc finger nuclease or a TALE-nuclease. In a preferred embodiment, said rare-cutting endonuclease is a TALE-nuclease. Preferred methods and relevant TALE-nucleases have been described in WO2013176915.

### Activation and expansion of T cells

Whether prior to or after genetic modification of the T cells, the T cells can be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005. T cells can be expanded *in vitro* or *in vivo.*

Generally, the T cells of the invention are expanded by contact with an agent that stimulates a CD3 TCR complex and a co-stimulatory molecule on the surface of the T cells to create an activation signal for the T-cell.

For example, chemicals such as calcium ionophore A23187, phorbol 12-myristate 13-acetate (PMA), or mitogenic lectins like phytohemagglutinin (PHA) can be used to create an activation signal for the T-cell.

As non limiting examples, T cell populations may be stimulated *in vitro* such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody. For example, the agents providing each signal may be in solution or coupled to a surface. As those of ordinary skill in the art can readily appreciate, the ratio of particles to cells may depend on particle size relative to the target cell. In further embodiments of the present invention, the cells, such as T cells, are combined with agent-coated beads, the beads and the cells are subsequently separated, and then the cells are cultured. In an alternative embodiment, prior to culture, the agent-coated beads and cells are not separated but are cultured together. Conditions appropriate for T cell culture include an appropriate media (e.g., Minimal Essential Media or RPMI Media 1640 or, X-vivo 5, (Lonza)) that may contain factors necessary for proliferation and viability, including serum (e.g., fetal bovine or human serum), interleukin-2 (IL-2), insulin, IFN-g, 1L-4, 1L-7, GM-CSF, - 10, - 2, 1L-15, TGFp, and TNF- or any other additives for the growth of cells known to the skilled artisan. Other additives for the growth of cells include, but are not limited to, surfactant, plasmanate, and reducing agents such as N-acetyl-cysteine and 2-mercaptoethanoi. Media can include RPMI 1640, A1M-V, DMEM, MEM, a-MEM, F-12, X-Vivo 1, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% C02). T cells that have been exposed to varied stimulation times may exhibit different characteristics
In another particular embodiment, said cells can be expanded by co-culturing with tissue or cells. Said cells can also be expanded *in vivo,* for example in the subject's blood after administrating said cell into the subject. Therapeutic applications

In another embodiment, isolated cell obtained by the different methods or cell line derived from said isolated cell as previously described can be used as a medicament.

The present invention provides an isolated immune cell expressing an anti- CS1 CAR according to the invention, for its use as a medicament.

Said isolated cell according to the above may be derived from, NK cells, inflammatory T-lymphocytes, cytotoxic T-lymphocytes, regulatory T-lymphocytes or helper T-lymphocytes.

The present invention provides an isolated immune cell derived from, NK cells, inflammatory T-lymphocytes, cytotoxic T-lymphocytes, regulatory T-lymphocytes or helper T-lymphocytes endowed with an anti-CS1 msCAR for its use as a medicament.

### COMPOSITION

The present invention provides a composition comprising at least one pharmaceutically acceptable vehicle and an isolated immune T cell said cell as above.

Preferably, the present invention provides a composition comprising at least one pharmaceutically acceptable vehicle and an isolated immune T cell said cell expressing at least one anti-CS1 CAR, preferably a CS1 specific multi-chain Chimeric Antigen Receptor (mc CAR) comprising:
A transmembrane polypeptide from the alpha chain of high-affinity IgE receptor (FcεRI) fused to an extracellular CS1 ligand binding domain;
A second transmembrane polypeptide from the gamma or beta chain of FcεRI fused to a signal transducing domain;
A third transmembrane polypeptide from the gamma or beta chain of FcεRI comprising a co-stimulatory domain.

The present invention provides a composition as above comprising at least one pharmaceutically acceptable vehicle and an isolated immune T cell as above expressing at least one CS1 specific multi-chain Chimeric Antigen Receptor (mc CAR), wherein a CS1 ligand binding domain is fused to an alpha chain of FCERI and is a single-chain variable fragment (scFv) comprising a heavy (V_{H}) and a light (V_{L}) chain conferring specificity to CS1.

The present invention provides a composition as above comprising at least one pharmaceutically acceptable vehicle and an isolated immune T cell as above expressing at least one CS1 specific multi-chain Chimeric Antigen Receptor (mc CAR), wherein said CS1 ligand binding domain fused to said alpha chain of FCεRI is a humanized single-chain variable fragment (scFv) comprising a heavy (V_{H}) and a light (V_{L}) chains conferring specificity to CS1.

The present invention provides a composition as above comprising at least one pharmaceutically acceptable vehicle and an isolated immune T cell as above expressing at least one CS1 specific multi-chain Chimeric Antigen Receptor (mc CAR), wherein said V_{H} comprises a polypeptide sequence displaying at least 80%, at least 81%, at least 82% at least 83% at least 84% at least 85% at least 86% at least 87% at least 88% at least 89% at least 90% at least 91% at least 92% at least 93% at least 94% at least 95% at least 96% at least 97% at least 98% at least 99% or 100 % identity to one selected from SEQ ID NO. 13, SEQ ID NO. 15, SEQ ID NO. 17, SEQ ID NO. 19 and SEQ ID NO. 21.

The present invention provides a composition as above comprising at least one pharmaceutically acceptable vehicle and an isolated immune T cell as above expressing at least one CS1 specific multi-chain Chimeric Antigen Receptor (mc CAR), wherein said V_{L} comprises a polypeptide displaying at least 80%, at least 81%, at least 82% at least 83% at least 84% at least 85% at least 86% at least 87% at least 88% at least 89% at least 90% at least 91% at least 92% at least 93% at least 94% at least 95% at least 96% at least 97% at least 98% at least 99% or 100 % identity to one selected from SEQ ID NO. 14, SEQ ID NO. 16, SEQ ID NO. 18, SEQ ID NO. 20 and SEQ ID NO. 22.
The present invention provides a method for treating a patient in need thereof comprising:
Providing a composition as above comprising an immune cell obtainable by a method according to the above;
Administrating said composition comprising aT-cells to said patient.
The present invention provides a method for treating a patient as above wherein said immune cells are recovered from donors and part of a composition as above.

The present invention provides a method for treating a patient as above wherein said immune cells are recovered from patients and part of a composition as above.

In one embodiment, the present invention provides a composition as above comprising at least one pharmaceutically acceptable vehicle and an isolated immune T cell as above expressing at least one CS1 specific multi-chain Chimeric Antigen Receptor (mc CAR) as above for use as a medicament to treat a disease or a complication related to said disease.

Preferably said disease may be treated using a composition according to the invention comprising an isolated immune T cell as above expressing at least one CS1-L specific multi-chain Chimeric Antigen Receptor (mc CAR) as above.

In another embodiment, said disease may be treated using a composition according to the invention comprising an isolated immune T cell as above expressing at least one CS1-S specific multi-chain Chimeric Antigen Receptor (mc CAR) as above.

In a preferred embodiment, a composition according to the invention is provided for use as a medicament.

In another embodiment, said medicament can be used for treating cancer or infections in a patient diagnosed with a pathology linked to CS1 positive cells.

In another embodiment, said isolated cell according to the invention or cell line derived from said isolated cell can be used in the manufacture of a medicament for treatment of a cancer, especially multiple myeloma.

In one embodiment, the present invention provides a composition comprising at least one pharmaceutically acceptable vehicle and an isolated immune T cell as above expressing at least one CS1 specific multi-chain Chimeric Antigen Receptor (mc CAR) as above for use as a medicament to treat a disease or a complication related to said disease.

In one embodiment said disease that may be treated using a composition according to the invention is an auto immune disease, preferably an autoimmune inflammatory disease, for example Systemic lupus erythematosus (SLE) or inflammatory bowel disease (IBD).

In one embodiment said disease that may be treated using a composition according to the invention is a cancer, a hematological cancer for example Multiple myeloma (MM).

In another aspect, the present invention relies on methods for treating patients in need thereof, said method comprising at least one of the following steps:
(a) providing an immune-cell obtainable by any one of the methods previously described;
(b)Administrating said transformed immune cells to said patient,
On one embodiment, said T cells of the invention can undergo robust *in vivo* T cell expansion and can persist for an extended amount of time.

Said treatment can be ameliorating, curative or prophylactic. It may be either part of an autologous immunotherapy or part of an allogenic immunotherapy treatment. By autologous, it is meant that cells, cell line or population of cells used for treating patients are originating from said patient or from a Human Leucocyte Antigen (HLA) compatible donor. By allogeneic is meant that the cells or population of cells used for treating patients are not originating from said patient but from a donor.

The invention is particularly suited for allogenic immunotherapy, insofar as it enables the transformation of T-cells, typically obtained from donors, into non-alloreactive cells. This may be done under standard protocols and reproduced as many times as needed. The resulted modified T cells may be pooled and administrated to one or several patients, being made available as an "off the shelf" therapeutic product.

Cells that can be used with the disclosed methods are described in the previous section. Said treatment can be used to treat patients diagnosed with cancer, viral infection, autoimmune disorders or Graft versus Host Disease (GvHD).

In a preferred embodiment said treatment comprising an engineered T cell expressing an anti-CS1 CAR of the invention can be used to treat patients diagnosed with autoimmune disorders, preferably SLE or IBD.

In a more preferred embodiment, said treatment comprising an engineered T cell expressing an anti-CS1 CAR of the invention can be used to treat patients diagnosed with Multiple Myeloma

Cancers that may be treated include tumors that are not vascularized, or not yet substantially vascularized, as well as vascularized tumors. The cancers may comprise nonsolid tumors (such as hematological tumors, for example, leukemias and lymphomas) or may comprise solid tumors. Types of cancers to be treated with the multi-chain CARs of the invention include, but are not limited to, carcinoma, blastoma, and sarcoma, and certain leukemia or lymphoid malignancies, benign and malignant tumors, and malignancies e.g., sarcomas, carcinomas, and melanomas, in particular Multiple Myeloma. Adult tumors/cancers and pediatric tumors/cancers are also included.

In a preferred embodiment, patients who can benefit from a treatment comprising an engineered T cell expressing an anti-CS1 CAR of the invention are suffering from MM, relapse or refractory MM or a complication related to MM.

MM can be ranging from monoclonal gammopathy of unknown significance (MGUS) to plasma cell leukemia.

COMBINATION THERAPY It can be a treatment in combination with one or more therapies against cancer selected from the group of antibodies therapy, chemotherapy, cytokines therapy, dendritic cell therapy, gene therapy, hormone therapy, laser light therapy and radiation therapy.

In one embodiment, the present invention provides a combination of an engineered T cell expressing an anti-CS1 CAR of the invention and an anti-cancer drug as a treatment for MM.

Preferably, a T cell expressing an anti-CS1 CAR is provided which can survive and proliferate in the present of the anti-cancer drug with which it is combined to.

An anti-cancer drug can be a treatment used to treat MM, relapsed MM or refractory MM, for example.

In a preferred embodiment the present invention provides a combination of an engineered T cell expressing an anti-CS1 msCAR of the invention and at least one of the following treatments : Thalidomide, either as a single agent or in combination with a steroid or with melphalan, Lenalidomide plus dexamethasone, Bortezomib plus melphalan, VAD (vincristine, doxorubicin [Adriamycin] and dexamethasone, Melphalan plus prednisone.
For primary induction therapy in transplant candidates the following combination therapies are associated with the object of the present invention Bortezomib/dexamethasone, Bortezomib/doxorubicin/dexamethasone, Bortezomib/thalidomide/dexamethasone, Lena lidomide/dexa methasone.
The following combinations in association with a T cell expressing an anti-CS1 CAR of the invention are preferred for primary induction therapy in patients who are not transplant candidates, Lenalidomide/ dexamethasone, Melphalan/prednisone/bortezomib (MPB), Melphalan/prednisone/lenalidomide (MPL), Melphalan/prednisone/thalidomide (MPT).
Patients with refractory disease or relapse may be treated with a T cell expressing an anti-CS1 CAR of the invention and the following:
Any of the agents not previously used, Bortezomib plus cyclophosphamide and dexamethasone, Carfilzomib (Kyprolis), Thalidomide, Lenalidomide plus cyclophosphamide and dexamethasone, Pomalidomide.

In a preferred embodiment, the present invention provides a combination of an engineered T cell expressing an anti-CS1 CAR of the invention and another engineered T cell expressing an anti-CD38 CAR and or an anti-IRF4 CAR as a treatment for refractory MM.

According to a preferred embodiment of the invention, said treatment can be administrated into patients undergoing an immunosuppressive treatment. Indeed, the present invention preferably relies on cells or population of cells, which have been made resistant to at least one immunosuppressive agent due to the inactivation of a gene encoding a receptor for such immunosuppressive agent. In this aspect, the immunosuppressive treatment should help the selection and expansion of the T-cells according to the invention within the patient.

The administration of the cells or population of cells according to the present invention may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous or intralymphatic injection, or intraperitoneally. In one embodiment, the cell compositions of the present invention are preferably administered by intravenous injection.

The administration of the cells or population of cells can consist of the administration of 10⁴-10⁹ cells per kg body weight, preferably 10⁵ to 10⁶ cells/kg body weight including all integer values of cell numbers within those ranges. The cells or population of cells can be administrated in one or more doses. In another embodiment, said effective amount of cells are administrated as a single dose. In another embodiment, said effective amount of cells are administrated as more than one dose over a period time. Timing of administration is within the judgment of managing physician and depends on the clinical condition of the patient. The cells or population of cells may be obtained from any source, such as a blood bank or a donor. While individual needs vary, determination of optimal ranges of effective amounts of a given cell type for a particular disease or conditions within the skill of the art. An effective amount means an amount which provides a therapeutic or prophylactic benefit. The dosage administrated will be dependent upon the age, health and weight of the recipient, kind of concurrent treatment, if any, frequency of treatment and the nature of the effect desired.

In another embodiment, said effective amount of cells or composition comprising those cells are administrated parenterally. Said administration can be an intravenous administration. Said administration can be directly done by injection within a tumor.

In certain embodiments of the present invention, cells are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efaliztimab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycoplienolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin) (Liu et al., Cell 66:807-815, 1 1; Henderson et al., Immun. 73:316-321, 1991; Bierer et al., Citrr. Opin. mm n. 5:763-773, 93). In a further embodiment, the cell compositions of the present invention are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH, In another embodiment, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery. Said modified cells obtained by any one of the methods described here can be used in a particular aspect of the invention for treating patients in need thereof against Host versus Graft (HvG) rejection and Graft versus Host Disease (GvHD); therefore in the scope of the present invention is a method of treating patients in need thereof against Host versus Graft (HvG) rejection and Graft versus Host Disease (GvHD) comprising treating said patient by administering to said patient an effective amount of modified cells comprising inactivated TCR alpha and/or TCR beta genes.

### RESISTANCE to anti-chemotherapy drug

### Drug Resistant T-cells

According to one aspect, the anti-CS1 ms CAR expressing T-cell of the invention can be further genetically engineered to improve its resistance to immunosuppressive drugs or chemotherapy treatments, which are used as standard care for treating CS1 positive malignant cells.

To improve cancer therapy and selective engraftment of allogeneic T-cells, drug resistance is conferred to said allogeneic T cells to protect them from the toxic side effects of chemotherapy agent. The drug resistance of T-cells also permits their enrichment *in* or *ex vivo*, as T-cells which express the drug resistance gene will survive and multiply relative to drug sensitive cells.

Methods for engineering T-cells resistant to chemotherapeutic agents are disclosed in PCT/EP2014/075317 which is fully incorporated by reference herein.

In particular, the present invention relates to a method of engineering immune cells suitable for immunotherapy wherein at least one gene encoding a T-cell receptor (TCR) component is inactivated and one gene is modified to confer drug resistance comprising:
∘ Providing an anti-CS1 ms CAR expressing T-cell;
∘ Modifying said anti-CS1 msCAR expressing T-cell by inactivating at least one gene encoding a T-cell receptor (TCR) component;
∘ Modifying said anti-CS1 msCAR expressing T-cell to confer drug resistance to said anti-CS1 msCAR expressing T-cell;
∘ Expanding said engineered anti-CS1 msCAR expressing T-cell in the presence of said drug.

Alternatively, the present invention relates to a method comprising:
∘ Providing an anti-CS1 msCAR expressing T-cell;
∘ Modifying said anti-CS1 msCAR expressing T-cell to confer drug resistance to said anti-CS1 msCAR expressing T-cell;
∘ Modifying said anti-CS1 msCAR expressing T-cell by inactivating at least one gene encoding a T-cell receptor (TCR) component;
∘ Expanding said engineered anti-CS1 ms CAR expressing T-cell in the presence of said drug.

In particular, the present invention also relates to a method of engineering immune cells suitable for immunotherapy wherein at least one gene encoding a T-cell receptor (TCR) component is inactivated and one gene is modified to confer drug resistance comprising:
∘ Providing an anti-CS1 msCAR expressing T-cell;
∘ Modifying said anti-CS1 msCAR expressing T-cell by inactivating at least one gene encoding a T-cell receptor (TCR) component;
∘ Modifying said anti-CS1 msCAR expressing T-cell to confer drug resistance to said anti-CS1 msCAR expressing T-cell;
∘ Expanding said engineered anti-CS1 msCAR expressing T-cell in the presence of said drug.

Alternatively, the present invention relates to a method comprising:
∘ Providing an anti-CS1 msCAR expressing T-cell;
∘ Modifying said anti-CS1 msCAR expressing T-cell to confer drug resistance to said anti-CS1 msCAR expressing T-cell;
∘ Modifying said anti-CS1 msCAR CAR expressing T-cell by inactivating at least one gene encoding a T-cell receptor (TCR) component;
∘ Expanding said engineered anti-CS1 msCAR expressing T-cell in the presence of said drug.

### Expression of drug resistance genes in anti-CS1 ms CAR-expressing immune cells

In a particular embodiment, said drug resistance can be conferred to the T-cell by the expression of at least one drug resistance gene. Said drug resistance gene refers to a nucleic acid sequence that encodes "resistance" to an agent, such as a chemotherapeutic agent (e.g. bortezomib, see Lü and Wang. Biomarker Research 2013,1:13).

In other words, the expression of the drug resistance gene in a cell permits proliferation of the cells in the presence of the agent to a greater extent than the proliferation of a corresponding cell without the drug resistance gene. The expression of the drug resistance gene in a cell permits proliferation of the cells in the presence of the agent and does not affect its activity.

In one embodiment, a drug resistance gene of the invention can confer resistance to a drug (or an agent), in particular an anti-cancer drug selected from and combination thereof.

In a preferred embodiment, cells bearing such a drug resistance conferring mRNA or protein also comprise an inhibitory mRNA or a gene the expression of which is conditioned, allowing the selective destruction of said drug resistant cells in the presence of said drug or upon administration of said drug.

### - Suicide genes in anti-CS1 ms CAR-expressing immune cells

- In one embodiment, cells bearing a drug resistance gene or a modified gene conferring resistance to a drug also comprise an inducible suicide gene - the induction of which provokes cell death- allowing their selective destruction.

In some instances, since engineered T-cells can expand and persist for years after administration, it can be desirable to include a safety mechanism to allow selective deletion of administrated T-cells. Thus, in some embodiments, the method of the invention can comprises the transformation of said T-cells with a recombinant suicide gene. Said recombinant suicide gene is used to reduce the risk of direct toxicity and/or uncontrolled proliferation of said T-cells once administrated in a subject (Quintarelli C, Vera F, blood 2007; Tey SK, Dotti G. , Rooney CM, boil blood marrow transplant 2007). Suicide genes enable selective deletion of transformed cells *in vivo.* In particular, the suicide gene has the ability to convert a non-toxic pro-drug into cytotoxic drug or to express the toxic gene expression product. In other words, "Suicide gene" is a nucleic acid coding for a product, wherein the product causes cell death by itself or in the presence of other compounds.

A representative example of such a suicide gene is one which codes for thymidine kinase of herpes simplex virus. Additional examples are thymidine kinase of varicella zoster virus and the bacterial gene cytosine deaminase which can convert 5-fluorocytosine to the highly toxic compound 5-fluorouracil. Suicide genes also include as non limiting examples caspase-9 or caspase-8 or cytosine deaminase. Caspase-9 can be activated using a specific chemical inducer of dimerization (CID). Suicide genes can also be polypeptides that are expressed at the surface of the cell and can make the cells sensitive to therapeutic monoclonal antibodies. As used herein "prodrug" means any compound useful in the methods of the present invention that can be converted to a toxic product. The prodrug is converted to a toxic product by the gene product of the suicide gene in the method of the present invention. A representative example of such a prodrug is ganciclovir which is converted in vivo to a toxic compound by HSV-thymidine kinase. The ganciclovir derivative subsequently is toxic to tumor cells. Other representative examples of prodrugs include acyclovir, FIAU [1-(2-deoxy-2-fluoro-β-D-arabinofuranosyl)-5-iodouracil], 6-methoxypurine arabinoside for VZV-TK, and 5-fluorocytosine for cytosine deaminase.

One preferred suicide gene system employs a recombinant antigenic polypeptide comprising antigenic motif recognized by the anti-CD20 mAb Rituximab, especially QBen10, such as in the so-called RQR8 polypeptide described in WO2013153391. Rituximab, an authorized antibody drug, can then be used for cell depletion when needed.

In one embodiment, the present invention provides anti-CS1 ms CAR expressing T-cell comprising
at least one drug resistance gene or wherein at least one drug sensitizing gene is inactivated, and a suicide gene, preferably RQR8 allowing said cells to be destroyed.

The random insertion of genes into the genome may lead to the inappropriate expression of the inserted gene or the gene near the insertion site. Specific gene therapy using homologous recombination of exogenous nucleic acid comprising endogenous sequences to target genes to specific sites within the genome can allow engineering secure T-cells. As described above, the genetic modification step of the method can comprise a step of introduction into cells of an exogeneous nucleic acid comprising at least a sequence encoding the drug resistance gene and a portion of an endogenous gene such that homologous recombination occurs between the endogenous gene and the exogeneous nucleic acid. In a particular embodiment, said endogenous gene can be the wild type "drug resistance" gene, such that after homologous recombination, the wild type gene is replaced by the mutant form of the gene which confers resistance to the drug.

### - METHODPrimary T-cell cultures

T cells are purified from Buffy coat samples provided by for example EFS (Etablissement Français du Sang, Paris, France) using Ficoll gradient density medium. The PBMC layer is recovered and T cells are purified using a commercially available T-cell enrichment kit. Purified T cells are activated in X-Vivo™-15 medium (Lonza) supplemented with 20ng/mL Human IL-2, 5% Human, and Dynabeads Human T activator CD3/CD28 at a bead:cell ratio 1:1 (Life Technologies).

### - CAR mRNA transfection

Transfections are performed at day 4 or day 11 after T-cell purification and activation. 5 millions of cells are transfected with 15µg of mRNA encoding the different parts of the CAR constructs. CAR mRNAs are produced using T7 mRNA polymerase transfections done using Cytopulse technology, by applying two 0.1 mS pulses at 3000V/cm followed by four 0.2 mS pulses at 325V/cm in 0.4cm gap cuvettes in a final volume of 200µl of "Cytoporation buffer T" (BTX Harvard Apparatus). Cells are immediately diluted in X-Vivo™-15 media and incubated at 37°C with 5% CO₂. IL-2 is added 2h after electroporation at 20ng/mL.

### - Degranulation assay (CD107a mobilization)

T-cells are incubated in 96-well plates (40,000 cells/well), together with an equal amount of cells expressing various levels of the ms CAR of the invention. Co-cultures are maintained in a final volume of 100µl of X-Vivo™-15 medium (Lonza) for 6 hours at 37°C with 5% CO₂. CD107a staining was done during cell stimulation, by the addition of a fluorescent anti-CD107a antibody at the beginning of the co-culture, together with 1µg/ml of anti-CD49d, 1µg/ml of anti-CD28, and 1x Monensin solution. After the 6h incubation period, cells are stained with a fixable viability dye and fluorochrome-conjugated anti-CD8 and analyzed by flow cytometry. The degranulation activity was determined as the % of CD8+/CD107a+ cells, and by determining the mean fluorescence intensity signal (MFI) for CD107a staining among CD8+ cells. Degranulation assays were carried out 24h after mRNA transfection.

### - IFN gamma release assay

T-cells are incubated in 96-well plates (40,000 cells/well), together with cell lines expressing various levels of the CS1 protein. Co-cultures were maintained in a final volume of 100µl of X-Vivo™-15 medium (Lonza) for 24 hours at 37°C with 5% CO₂. After this incubation period the plates were centrifuged at 1500 rpm for 5 minutes and the supernatants were recovered in a new plate. IFN gamma detection in the cell culture supernatants was done by ELISA assay. The IFN gamma release assays are carried by starting the cell co-cultures 24h after mRNA transfection.

### - Cytotoxicity assay

T-cells are incubated in 96-well plates (100,000 cells/well), together with 10,000 target cells (expressing CS1) and 10,000 control (CS1 neg) cells in the same well. Target and control cells are labelled with fluorescent intracellular dyes (CFSE or Cell Trace Violet) before co-culturing them with msCAR+ T-cells. The co-cultures are incubated for 4 hours at 37°C with 5% CO₂. After this incubation period, cells are labelled with a fixable viability dye and analyzed by flow cytometry. Viability of each cellular population (target cells or CSlneg control cells) was determined and the % of specific cell lysis was calculated. Cytotoxicity assays were carried out 48h after mRNA transfection.

### - T-cell transduction

Transduction of T-cells with recombinant lentiviral vectors expression the CAR is carried out three days after T-cell purification/activation. CAR detection at the surface of T-cells is done using a recombinant protein consisting on the fusion of the extracellular domain of the human CS1 protein, together with a murine IgG1 Fc fragment. Binding of this protein to the CAR molecule is detected with a fluorochrome-conjugated secondary antibody targeting the mouse Fc portion of the protein, and analyzed by flow cytometry.

### - Anti-tumor mouse model

Immunodefficient NOG mice are intravenously (iv) injected with CS1 -Luciferase expressing cells as an MM xenograft mouse model. Optionally, mice receive an anti-cancer treatment for example, BORTEZOMIB. Mice are then iv injected (either 2 or 7 days after injection of the tumor cell line) with different doses of CAR+ T-cells to be tested, or with T-cells that were not transduced with the CAR lentiviral vector. Bioluminescent signals are determined at the day of T-cell injection (D0), at D7, 14, 21, 28 and 40 after T-cell injection in order to follow tumoral progression on the different animals.

### CS1+/luc+ drug resistant NCI-H929 cells for testing the cytotoxicity of drug resistant allogenic CART cells.

The present invention encompasses a method for manufacturing a target cell line which express both a surface receptor specific to the CAR T cells (CS1) and a resistance gene. These target cells are particularly useful for testing the cytotoxicity of CAR T cells of the invention. These cells are readily resistant to clinically relevant dose of drugs used against MM and harbor luciferase activity. This combination of features enable traking them *in vivo* in a mice model or destroy them when required.

More particularly, they can be used to assess the cytotoxicity properties drug resistant T cells in mice in the presence of a chemotherapy or a combination thereof. Bortezomib resistant NCI-H929 cells mimick the physiological state of MM patients, that may harbor drug resistant B cell malignancies. Thus, these cells are of great interest to evaluate the reliability and cytotoxicity of drug resistant CAR T cells. Preferably, these target cells are CS1+ Luciferase+ H929 cells. Other models of CS1+/luc+ drug resistant are provided using cells expressing various level of CS1 (IM9, MM.1S and RPMI-8226). Cells may be engineered to modulate drug resistance and allow their suicide.

### Example of CS1 specific multi-chain CARs

### A. Design of multi-chain CARs

Ten multi-chain CARs targeting the CS1 antigen were designed based on the high affinity receptor for IgE (FcεRI). The FcεRI expressed on mast cells and basophiles triggers allergic reactions. It is a tetrameric complex composed of a single α subunit, a single β subunit and two disulfide-linked γ subunits. The α subunit contains the IgE-binding domain. The β and γ subunits contain ITAMs that mediate signal transduction. In every multi-chain CAR, the extracellular domain of the FcRα chain was deleted and replaced by the respective scFv referred to µln Table 5 respectively and the CD8α hinge (SEQ ID NO: 2) and the ITAM of the FcRβ chain and/or the FcRγ chain was deleted. The resulting constructions had the structure detailed in table 6.

### B. Transiently expression in T cells

### Multi-chain CARs can be expressed in human T cells after electroporation of polycistronic mRNA.

T cells were electroporated with capped and polyadenylated polycistronic mRNA that were produced using the mMESSAGE mMACHINE kit and linearized plasmids as template. The plasmids used as template contained the T7 RNA polymerase promoter followed by a polycistronic DNA sequence encoding the different CAR variants.

The electroporation of the polycistronic mRNAs into the human T cells was done using the CytoLVT-S device (Cellectis), according to the following protocol: 5X10⁶ T cells preactivated several days (3-5) with anti CD3/CD28 coated beads and IL2 were resuspended in cytoporation buffer T, and electroporated in 0.4cm cuvettes with 45µg of mRNA using the PBMC3 program Table 14.

24 hours post electroporation, human T cells engineered using polycistronic mRNAs encoding the multi-chain CARs were labeled with a fixable viability dye eFluor-780 and a PE-conjugated goat anti mouse IgG F(ab')2 fragment specific, and analysed by flow cytometry.

The live T cells engineered using polycistronic mRNAs expressed the multi-chain CARs on their surface.

### C. The human T cells transiently expressing the multi-chain CARs degranulate following coculture with target cells

24 hours post electroporation, human T cells engineered using polycistronic mRNAs encoding the multi-chain CARs were co-cultured with target (Daudi) or control (K562) cells for 6 hours. The CD8+ T cells were then analyzed by flow cytometry to detect the expression of the degranulation marker CD107a at their surface. The data indicate that the human CD8+ T cells expressing the CS1 multi-chain CARs degranulate in coculture with CS1 expressing target cells but not in coculture with control cells.

### D. The human T cells transiently expressing the multi-chain CARs secrete cytokines following coculture with target cells

24 hours post electroporation, human T cells engineered using polycistronic mRNAs encoding the multi-chain CARs were co-cultured with target (Daudi) or control (K562) cells for 24 hours. The supernatants were then harvested and analysed using the TH1/TH2 cytokine cytometric bead array kit to quantify the cytokines produced by the T cells. The assay indicated that the human T cells expressing the multi-chain CARs produce IFNγ, IL8 and IL5 in coculture with CS1 expressing target cells but not in coculture with control cells.

### E. The human T cells transiently expressing the multi-chain CARs lyse target cells

24 hours post electroporation, human T cells engineered using polycistronic mRNAs encoding the multi-chain CARs were co-cultured with target (Daudi) or control (K562) cells for 4 hours. The target cells were then analyzed by flow cytometry to analyze their viability. indicating that the different cells expressing the CS1 multi-chain CARs lyse the CS1 expressing target cells but not the control cells.

### Further embodiments

1) A CS1 specific multi-chain Chimeric Antigen Receptor (mc CAR) comprising:
   - A transmembrane polypeptide from the alpha chain of high-affinity IgE receptor (FcεRI) fused to an extracellular CS1 ligand binding domain;
2) A CS1 specific multi-chain Chimeric Antigen Receptor (mc CAR) according to item 1 further comprising :
   - A second transmembrane polypeptide from the gamma or beta chain of FcεRI fused to a signal transducing domain;
3) A CS1 specific multi-chain Chimeric Antigen Receptor (mc CAR) according to item 2, further comprising :
   - A third transmembrane polypeptide from the gamma or beta chain of **FcεRI** comprising a co-stimulatory domain.
4) A CS1 specific multi-chain Chimeric Antigen Receptor according to any one of items 1 to 3, wherein said CS1 ligand binding domain fused to said alpha chain of FcεRI is a single-chain variable fragment (scFv) comprising heavy (V_{H}) and light (V_{L}) chains conferring specificity to CS1.
5) A CS1 specific multi-chain Chimeric Antigen Receptor of item 4, wherein said V_{H} comprises a polypeptide sequence displaying at least 90 % identity to one selected from SEQ ID NO. 13, SEQ ID NO. 15, SEQ ID NO. 17, SEQ ID NO. 19 and SEQ ID NO. 21.
6) A CS1 specific multi-chain Chimeric Antigen Receptor of item 1, wherein said V_{L} comprises a polypeptide displaying at least 90 % identity to one selected from SEQ ID NO. 14, SEQ ID NO. 16, SEQ ID NO. 18, SEQ ID NO. 20 and SEQ ID NO. 22.
7) A CS1 specific multi-chain Chimeric Antigen Receptor of item 1, wherein said alpha chain of FcεRI is fused to said extracellular ligand-binding domain by a hinge from CD8α, IgG1 or FcRIIIα proteins.
8) A CS1 specific multi-chain Chimeric Antigen Receptor of item 1, wherein said hinge comprises a polypeptide sequence displaying at least 90 % identity to SEQ ID NO.2.
9) A CS1 specific multi-chain Chimeric Antigen Receptor according to any one of items 2 to 8, wherein said signal transducing domain fused to the gamma or beta chain of FcεRI is from the TCR zeta chain, the FCεRβ chain, the FcεRIγ chain, or includes an immunoreceptor tyrosine-based activation motif (ITAM).
10) A CS1 specific multi-chain Chimeric Antigen Receptor according to item 9, wherein said signal transducing domain is from CD3zeta.
11) A CS1 specific multi-chain Chimeric Antigen Receptor according to item 10, wherein said signal transducing domain comprises a polypeptide sequence displaying at least 90 % identity to SEQ ID NO.10.
12) A CS1 specific multi-chain Chimeric Antigen Receptor according to any one of items 3 to 11, wherein said second or third polypeptide comprises a co-stimulatory domain from the cytoplasmic domain of a costimulatory molecule selected from CD27, CD28, 4-1BB, OX40, CD30, CD40, PD-1, ICOS, lymphocyte function-associated antigen-1 (LFA-1), CD2, CD7, CD8, LIGHT, NKG2C, B7-H3, a ligand that specifically binds with CD83, and any combination thereof.
13) A CS1 specific multi-chain Chimeric Antigen Receptor according to item 12, wherein said co-stimulatory domain is from 4-1BB and comprises a polypeptide sequence displaying at least 90 % identity to SEQ ID NO.6.
14) A CS1 specific multi-chain Chimeric Antigen Receptor according to item 12, wherein said co-stimulatory domain is from CD28 and comprises a polypeptide sequence displaying at least 90 % identity to SEQ ID NO.7.
15) A polypeptide encoding a CS1 specific multi-chain Chimeric Antigen Receptor according to item 1, comprising a polypeptide sequence displaying at least 80 % identity to the full amino acid sequence of CS1-Luc63, CS1-Luc90, CS1-Luc34 or CS1-Luc63 as referred to in Table 6.
16) A polynucleotide comprising a nucleic acid sequence encoding a CS1 specific multi-chain Chimeric Antigen Receptor according to any one of items 1 to 15.
17) A vector comprising a polynucleotide of item 16.
18) A method of engineering an immune cell comprising:
   (c) Providing an immune cell;
   (d) Expressing at the surface of said cells at least one multi-chain Chimeric Antigen Receptor according to any one of the items 1 to 15.
19) The method of engineering an immune cell of item 18 comprising:
   (d) Providing an immune cell;
   (e) Introducing into said cell at least one polynucleotide encoding polypeptides composing at least one multi-chain Chimeric Antigen Receptor according to any one of items 1 to 15;
   (f) Expressing said polynucleotides into said cell.
20) The method of engineering an immune cell of item 18 comprising:
   (c) Providing an immune cell;
   (d) Expressing at the surface of said cell a population of multi-chain Chimeric Antigen Receptors according to any one of the items 1 to 15 each one comprising different extracellular ligand-binding domains.
21) The method of engineereing an immune cell of item 18 comprising:
   (d) Providing an immune cell;
   (e) Introducing into said cell at least one polynucleotide encoding polypeptides composing a population of multi-chain Chimeric Antigen Receptors according to any one of items 1 to 15 each one comprising different extracellular ligand binding domains.
   (f) Expressing said polynucleotides into said cell.
22) An isolated immune cell obtainable from the method according to any one of items 18 to 21.
23) An isolated immune cell comprising at least one multi-chain Chimeric Antigen Receptor according to any one of items 1 to 15.
24) An isolated immune cell according to item 22 or 23 for its use as a medicament.
25) An isolated cell according to any one of items 22 to 23 derived from, NK cells, inflammatory T-lymphocytes, cytotoxic T-lymphocytes, regulatory T-lymphocytes or helper T-lymphocytes.
26) A method for treating a patient in need thereof comprising:
   a) Providing an immune cell obtainable by a method according to any one of the items 18 to 21;
   b) Administrating said T-cells to said patient,
27) The method for treating a patient of item 26 wherein said immune cells are recovered from donors.
28) The method for treating a patient of item 26 wherein said immune cells are recovered from patients.

## Claims

1. An engineered T-cell expressing an CS1 specific Chimeric Antigen Receptor (CAR) for its use as a medicament in allogeneic immunotherapy treatment.

2. The engineered T-cell for use according to claim 1, wherein said immunotherapy is for treating patients diagnosed with Multiple Myeloma.

3. The engineered T-cell for use according to claim 1 or 2, wherein said T-cell is made available as an "off the shelf" therapeutic product.

4. The engineered T-cell for use according any one of claims 1 to 3, wherein TCR is rendered not functional in said T-cell by inactivating TCR alpha gene and/or TCR beta gene(s).

5. The engineered T-cell for use according any one of claims 1 to 4, wherein said T-cells is genetically engineered to inactivate one or several genes selected from the group consisting of CD52, GR, TCR alpha, TCR beta, HLA gene, immune check point genes such as PD1 and CTLA-4.

6. The engineered T-cell for use according any one of claims 1 to 5, wherein said T-cell is obtained from a donor.

7. The engineered T-cell for use according to any one of claims 1 to 6, wherein said CS1 specific CAR is a CS1 specific multi-chain CAR.

8. The engineered T-cell for use according to claim 7, wherein said CS1 specific multi-chain CAR comprises:
- a transmembrane polypeptide from the alpha chain of high-affinity IgE receptor (FcεRI) fused to an extracellular CS1 ligand binding domain;
- a second transmembrane polypeptide from the gamma or beta chain of FcεRI fused to a signal transducing domain; and optionally
- a third transmembrane polypeptide from the gamma or beta chain of FcεRI comprising a co-stimulatory domain.

9. The engineered T-cell for use according to claim 8, wherein said extracellular CS1 ligand binding domain is a single-chain variable fragment (scFv) comprising heavy (VH) and light (VL) chains conferring specificity to CS1.

10. The engineered T-cell for use according to claim 9, wherein said VH comprises a polypeptide sequence displaying at least 90 % identity to one selected from SEQ ID NO. 13, SEQ ID NO. 15, SEQ ID NO. 17, SEQ ID NO. 19 and SEQ ID NO. 21., and said VL comprises a polypeptide sequence displaying at least 90 % identity to one selected from SEQ ID NO. 14, SEQ ID NO. 16, SEQ ID NO. 18, SEQ ID NO. 20 and SEQ ID NO. 22.

11. The engineered T-cell for use according any one of claims 8 to 10, wherein said alpha chain of FcεRI is fused to said extracellular ligand-binding domain by a hinge from CD8α, IgG1 or FcRIIIα proteins.

12. The engineered T-cell for use according any one of claims 8 to 11, wherein said signal transducing domain fused to the gamma or beta chain of FcεRI is from CD3zeta.

13. The engineered T-cell according any one of claims 8 to 12, wherein said second or third polypeptide comprises a co-stimulatory domain from 4-1BB or CD28.

14. The engineered T-cell for use according any one of claims 1 to 13, wherein said T-cell further comprises a suicide gene system that employs a recombinant antigenic polypeptide comprising antigenic motif recognized by the anti-CD20 mAb Rituximab, especially QBen10.

15. The engineered T-cell for use according any one of claims 1 to 14, wherein said engineered T-cell is derived from a cytotoxic T-lymphocyte.
